(19) Europäisches Patentamt
European Patent Office
Office européen des brevets

(11) Veröffentlichungsnummer: **0 224 159**
**B1**

(12) # EUROPÄISCHE PATENTSCHRIFT

(45) Veröffentlichungstag der Patentschrift: **02.01.91**

(51) Int. Cl.[5]: **C 07 D 213/74,**
C 07 D 213/89,
C 07 D 213/80,
C 07 D 213/85,
C 07 D 401/12, A 61 K 31/44,
A 61 K 31/445, A 61 K 31/495

(21) Anmeldenummer: **86115896.2**

(22) Anmeldetag: **15.11.86**

(54) **Neue basisch substituierte Pyridinverbindungen, Verfahren zu ihrer Herstellung, die sie enthaltenden Arzneimittel und ihre Verwendung.**

(30) Priorität: **23.11.85 DE 3541428**

(43) Veröffentlichungstag der Anmeldung:
**03.06.87 Patentblatt 87/23**

(45) Bekanntmachung des Hinweises auf die Patenterteilung:
**02.01.91 Patentblatt 91/01**

(84) Benannte Vertragsstaaten:
**AT BE CH DE ES FR GB GR IT LI LU NL SE**

(56) Entgegenhaltungen:
**EP-A-0 135 087**
**DE-A-2 334 401**
**DE-A-2 900 504**

(73) Patentinhaber: **HOECHST AKTIENGESELLSCHAFT**
**Postfach 80 03 20**
**D-6230 Frankfurt am Main 80 (DE)**

(72) Erfinder: **Elben, Ulrich, Dr.**
**Eschenstrasse 23**
**D-6200 Wiesbaden (DE)**
Erfinder: **Anagnostopulos, Hiristo**
**Feldbergstrasse 6**
**D-6204 Taunusstein 02 (DE)**
Erfinder: **Bartlett, Robert R., Dr.**
**Sandbergstrasse 20**
**D-6100 Darmstadt (DE)**

**EP 0 224 159 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Eine im menschlichen Organismus durch Allergene ausgelöste allergische Reaktion vom sogenannten "Sofort-Typ" äußert sich bereits wenige Sekunden nach Allergenkontakt beispielsweise als Juckreiz (Dermatitis), Niesanfall (Rhinitis) oder Husten und Atemnot (Asthma). Diese Symptome werden durch die bei der Antigen-Antikörper-Reaktion aus den Gewebsmastzellen explosionsartig freigesetzten, spasmogen wirkenden Mediatoren wie Histamin, Bradykinin, PAF (platetlet activating factor), Leukotriene ($C_4$, $D_4$, $E_4$), Prostaglandine ($D_2$) und andere hervorgerufen, wobei die anaphylaktischen Antikörper der Immunglobulin-E-Klasse (IgE) angehören. Während die ersten drei der genannten Mediatoren in der Mastzelle in gespeicherter Form vorliegen, werden die Leukotriene und Prostaglandine erst im Verlaufe der anaphylaktischen Sofort-reaktion in der Zellmembran aus Arachidonsäure exzessiv gebildet.

Eine erfolgversprechende Allergietherapie muß demzufolge darauf abzielen, den kaskadenartig ablaufenden, selbstzerstörerischen Prozeß der IgE-vermittelten Immunreaktion an mindestens einem von mehreren möglichen Angriffspunkten wirksam zu unterbrechen. Hierfür bieten sich grundsätzlich zwei Therapieprinzipien an, nämlich die kausale und die symptomatische Behandlung. Die kausale Therapie besteht in der Expositionskarenz gegenüber dem jeweiligen Allergen oder in einer spezifischen Hyposensibilisierung des Allergikers. Nach klinischer Erfahrung sind jedoch nur bis zu 15% aller an exogen-allergischen Erkrankungen leidenden Patienten auf diese Weise behandelbar.

Im Vordergrund der Allergietherapie steht daher die symptomatische Behandlung mit Arzneimitteln, die entweder die Freisetzung der spasmogenen Mediatoren verhindern oder deren Wechselwirkung mit ihren spezifischen Rezeptoren unterbinden. Eine dominierende Rolle spielen hierbei die als $H_1$-Rezeptor-Antagonisten bekannten Antihistaminika. Da aber der Organismus nicht nur peripher, wie etwa im Brochialsystem, sondern auch im Zentralnervensystem mit $H_1$-Rezeptoren ausgestattet ist, führen nichtselectiv angreifende Antihistaminika über die gleichzeitige Blockade der zentralen Rezeptoren zu einer ausgeprägten Sedation (Müdigkeit), die als gravierende Nebenwirkung den täglichen Lebensablauf des Patienten stark beeinträchtigt. Eine klassisches Beispiel dafür ist die Verbindung Ketotifen.

Optimale Therapiechancen bei allergisch konditionierten Erkrankungen vom IgE-vermittelten Sofort-Typ bieten konsequenterweise solche Pharmaka, die die Freisetzung des Histamins und der anderen eingangs erwähten Allergiemediatoren nachhaltig hemmen, ausschließlich die peripheren $H_1$-Rezeptoren blockieren und gleichzeitig ihre Wirkung auch nach oraler Verabreichung sehr schnell entfalten.

Die DE—OS 29 00 504 (= US—PS 4 289 765) offenbart bronchospasmolytisch wirksame Pyridinverbindungen, die zwar antihistaminische Aktivität besitzen, aber — wie im pharmakologischen Versuchteil der vorliegenden Beschreibung durch Vergleichsuntersuchungen gezeigt wird — keine Wirkung gegen die IgE-vermittelte Freisetzung von Allergiemediatoren aus den Mastzellen aufweisen und demzufolge für die Therapie exogen-allergischer Erkrankungen nicht einsetzbar sind.

Die in der GB—PS 1 144 905 als Mastzellen stabilisierende Substanz beschriebene CROMOGLICINSÄURE kann zwar inhalativ, aber nicht oral und vor allem nur prophylaktisch angewendet werden. Die tricyclische Verbindung KETOTIFEN und das Benzimidazolon-Derivat OXATOMID verfügen zusätzlich über antagonistische Eingenschaften gegenüber Histamin und Leukotrienen, können jedoch nicht inhalativ verabreicht werden. Da OXATOMID seine Wirkung erst nach mehreren Tagen entfaltet, muß es vornehmlich der Behandlung von chronischen Allergiefällen vorbehalten bleiben. Darüber hinaus rufen beide Präparate eine ausgeprätge Sedation als unerwünschte Nebenwirkung bei Tier und Mensch hervor. Demgegenüber sollen das Butanol-Derivat TERFENADIN und die Benzimidazol-Verbindung ASTEMIZOL keine sedierende Nebenwirkung besitzen. Beide Präparate sind jedoch aufgrund ihrer Wasserunlöslichkeit nicht inhalativ anwendbar und führen wie auch andere Antihistaminika bei Dauertherapie zu einer unerwünschten Gewichtszunahme.

Für alle vorgenannten Wirkstoffe gilt, daß keine Injektions-lösungen vorliegen, die in akuten Situationen ein schnelles Eingreifen durch parenterale Applikation gestatten würden.

Überraschend wurde nun gefunden, daß durch Einführung bestimmter basisch funktionalisierter Alkylaminogruppen in die 4-Position von 3-substituierten 2,6-Dialkylpyridinen und deren 1-Oxiden neue Verbindungen erhalten werden, die sich aufgrund ihrer wertvollen pharmakologischen Eigenschaften sehr gut zur Behandlung allergisch konditionierter Erkrankungen vom IgE-vermittelten Sofort-Typ eignen. Sie zeigen eine ausgeprägte Hemmwirkung gegenüber den Spasmogenen Histamin, Bradykinin, dem plättchenaktivierenden Faktor (PAF) und den Leukotrienen, hemmen über ihren Mastzell-protektiven Effekt nachhaltig die Freisetzung dieser Allergiemediatoren, besitzen keine sedierende Nebenwirkung und können parenteral, inhalativ oder auch oral verabreicht werden, wobei ein schneller Wirkungseintritt gewährleistet ist. Sie besitzen folglich ein bislang unbekanntes optimales Wirkungsprofil und ermöglichen daher eine weitaus effektivere Therapie der allergischen Erkrankungen als die zum Stand der Technik gehörenden Präparate, die nur Teilaktivitäten aus dem oben beschriebenen idealen Wirkungsspektrum aufweisen.

Darüber hinaus eigenen sich die erfindungsgemäßen Verbindungen auch als Ausgangsstoffe für die Synthese weiterer wertvoller Pharmaka.

Die vorliegende Erfindung betrifft demzufolge neue basisch substituierte Pyridinverbindungen einschließlich der zugehörigen Salze, Verfahren zu deren Herstellung und deren Verwendung in Arzneimitteln, insbesondere in solchen, die bei atopischen Atemwegserkrankungen, wie allergischer

Rhinitis, allergischem Asthma und anaphylaktischem Schock, sowie bei allergischen Dermatitiden und allergischer Konjunktivitis indiziert sind.

Gegenstand der Erfindung sind somit die Pyridin-Derivate der Formel I

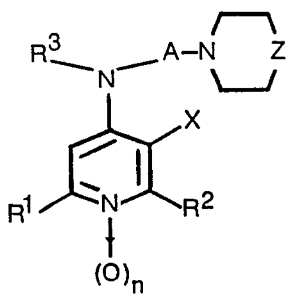

(I)

in der

$R^1$ und $R^2$ unabhängig voneinander für Wasserstoff oder Alkyl mit 1 bis 4 C-Atomen stehen,

$R^3$ Wasserstoff oder Alkyl mit 1 oder 2 C-Atomen und

A geradkettiges oder verzweigtes Alkylen mit 2 bis 4 C-Atomen bedeuten,

n den Wert 0 oder 1 hat,

Z eine Gruppe der Formel Za, Zb oder Zc

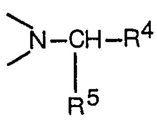 (Za) 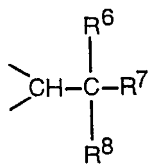 (Zb) 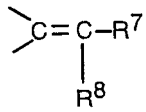 (Zc)

darstellt, wobei entweder

$R^4$ Wasserstoff und

$R^5$ Phenyl oder Cinnamyl oder

$R^4$, $R^5$, $R^7$ und $R^8$ unabhängig voneinander jeweils Pyridyl oder Phenyl, wobei das Phenyl gegebenenfalls einen oder zwei gleiche oder verschiedene Substituenten aus der Gruppe Halogen und Alkoxy mit bis zu 2 C-Atomen trägt,

$R^6$ Wasserstoff oder Hydroxy und

X Wasserstoff, eine Cyan-, Amino- oder Nitrogruppe oder den Rest —CO—$R^9$ bedeutet, in dem $R^9$ für Hydroxy oder Alkoxy mit 1 bis 4 C-Atomen steht,

und die physiologisch verträglichen Salze dieser Verbindungen.

Bevorzugt sind dabei solche Verbindungen der Formel I und deren Salze, bei denen die Reste $R^1$ und $R^2$ zusammen nicht mehr als 6 und insbesondere nicht mehr als 2 C-Atome enthalten und/oder das Halogen, sofern vorhanden, an den Phenylringen Fluor, Chlor oder Brom darstellt und/oder die Alkylenbrücke A Äthylen bedeutet. Unter diesen Verbindungen sind jene und deren Salze hervorzuheben, in denen $R^1$ und $R^2$ jeweils Methyl, $R^3$ Wasserstoff, A Äthylen, Z die Gruppe der Formel Za oder Zb und X die Cyan- oder Nitrogruppe bedeuten. Allgemein und besonders hierunter verdienen wiederum jene und deren Salze besonderes Interesse, bei denen Z die Gruppe der Formel Za darstellt und $R^4$ und $R^5$ die Bedeutung von Phenyl haben, das gegebenenfalls mit bis zu zwei gleichen oder verschiedenen Halogenatomen substituiert ist. In diese bevorzugt Verbindungsgruppe gehören insbesondere 3-Nitro- und 3-Cyan-2,6-dimethyl-4-[(2-{4-diphenylmethyl-1-piperazinyl}-äthyl)-amino]-pyridin und deren 1-Oxide sowie die Salze dieser Verbindungen.

Gegenstand der Erfindung ist weiterhin ein Verfahren zur Herstellung der Pyridinverbindungen gemäß Formel I und ihrer physiologisch verträglichen Salze, das dadurch gekennzeichnet ist, daß man

a) eine Verbindung der Formel II

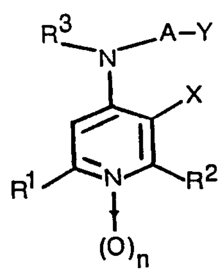

(II)

mit einem cyclischen Amin der Formel III

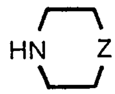

(III)

umsetzt, wobei $R^1$ bis $R^3$, n, X, A und Z mit $R^4$ bis $R^8$ die oben genannten Bedeutungen haben und Y für eine Abgangsgruppe wie Halogen, vorzugsweise Chlor, Brom oder Jod, eine Sulfonsäureester- oder Phosphorsäureester-Gruppierung steht, oder

b) eine Verbindungen der Formel IV

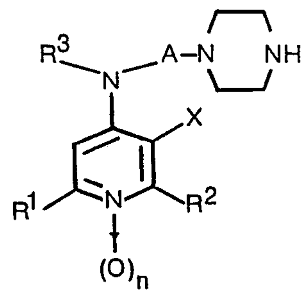

(IV)

in der $R^1$ bis $R^3$, n, X und A die vorgenannten Bedeutungen haben, mit einer Verbindungen der Formel V

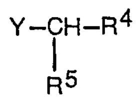

in der $R^4$, $R^5$ und Y die oben genannten Bedeutungen haben, zu einer Verbindung der Formel I mit dem Strukturelement Za umsetzt, oder

c) eine Verbindung der Formel VI

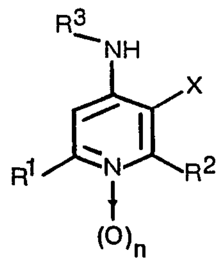

(VI)

in der $R^1$ bis $R^3$, n und X die vorgenannten Bedeutungen haben, mit einer Verbindung der Formel VII

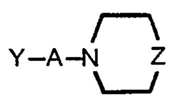

(VII)

umsetzt, wobei Y, A und Z mit $R^4$ bis $R^8$ die vorgenannten Bedeutungen haben, oder

d) eine Verbindung der Formel VIII

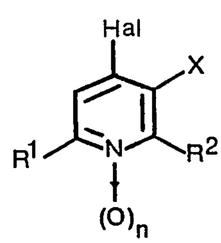

(VIII)

mit einem Amin der Formel IX

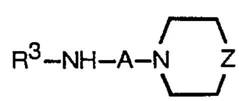

(IX)

umsetzt, wobei $R^1$ bis $R^3$, n, X, A und Z mit $R^4$ bis $R^8$ die oben definierten Bedeutungen haben und Hal ein Halogenatom darstellt,

daß man die Umsetzung gemäß den Verfahrensvarianten a), b), c) und d) in einem gegenüber den

Reaktionspartnern inerten Lösungs- oder Verteilungsmittel in Gegenwart eines Amin-Überschusses oder unter Zusatz eines anderen säurebindenden Mittels bei Temperaturen zwischen 0°C und dem Siedepunkt des jeweiligen Reaktionsmedium durchführt und das gemäß diesen Verfahrensvarianten erhaltene Produkt isoliert, wobei man

e) eine Verbindung der Formel I, in der X für die Aminogruppe steht, auch herstellen kann, was bevorzugt ist, indem man eine gemäß a), b), c) oder d) erhaltene 3-Nitroverbindung der Formel I vorzugsweise mit reduzierend wirkenden Metallsalzen oder durch katalytische Reduktion mit Hydrazin oder Wasserstoff in einem gegenüber den Reaktionsteilnehmern inerten Lösungs- oder Verteilungsmittel bei Temperaturen zwischen 0°C und 100°C bei Atmosphären- oder erhöhtem Druck reduziert, oder

f) eine Verbindung der Formel I, in der X die Carboxylgruppe bedeutet, auch herstellen kann, indem man einen gemäß a), b), c) oder d) erhaltenen 3-Carbonsäureester der Formel I, bevorzugt unter alkalischen Bedingungen, gegebenenfalls bei erhöhter Temperatur, hydrolysiert oder

g) eine Verbindung der Formel I mit dem Strukturmerkmal Zc auch herstellen kann, indem man eine gemäß a), b), c) oder d) erhaltene Verbindung der Formel I mit dem Strukturelement Zb, worin $R^6$ die Bedeutung von Hydroxy hat, in saurem Medium bei Temperaturen zwischen 0°C und dem Siedepunkt des jeweiligen Reaktionsgemisches, dehydratisiert,

wobei man die Verbindungen der Formel I entweder in freier Form isoliert oder sie mit geeigneten Säuren, oder für den Fall, daß X eine Carboxylgruppe darstellt, auch mit geeigneten Basen in physiologisch verträgliche Salze umwandelt.

Für die Herstellung von Säureadditionssalzen aus den Verbindungen der Formel I kommen beispielsweise Mineralsäuren, wie Schwefel- oder Phosphorsäure oder Halogenwasserstoffsäuren, insbesondere Salzsäuren, und organische Säuren, wie ein- bis dreibasische Carbonsäuren, z.B. Essig-, Milch-, Malein-, Fumar-, Oxal-, Wein-, Zitronen- oder Glukonsäure, oder andere physiologisch verträgliche Säuren, wie Sulfonsäuren, z.B. p-Toluolsulfon-, Methansulfon-, Trifluormethylsulfon- und Cyclohexylamidosulfonsäure in Frage.

Die Verbindungen der Formel I, in denen X eine Carboxylgruppe darstellt, können auch mit basischen Reagentien, wie Hydroxiden, Alkoholaten, Carbonaten und Hydrogencarbonaten, stabile wasserlösliche Alkali- und Erdalkalisalze bilden.

Die Ausgangsstoffe der Formeln II bis IX sind zumeist käuflich, literaturbekannt oder aber nach in der Literatur beschriebenen Methoden leicht herstellbar.

Geeignete Verbindungen der Formel II sind beispielsweise die aus der DE—OS 29 00 504 bekannten 3-substituierten, in 2,6-Stellung unsubstituierten, mono- oder dialkylierten 4-[N-(ω-Halogenalkyl)-amino]-pyridine, wie 4-[N-(2-Chloräthyl oder 3-Chlorpropyl)-amino]-2,6-dimethyl-3-nitropyridin, -3-cyanpyridin und -3-äthoxycarbonylpyridin und deren 2,6-Dipropyl-Derivate, 4-[N-(2-Chloräthyl oder 3-Chlorpropyl)-methylamino]-2,6-dimethyl (oder -dipropyl)-3-nitropyridin, -3-cyanpyridin und -3-äthoxycarbonylpyridin, sowie die 1-Oxide dieser Pyridinverbindungen, die sich durch N-Oxydation nach den in der DE—OS 35 14 073 beschriebenen Methoden gewinnen lassen.

Als cyclische Amine gemäß Formel III kommen unter anderen 1-Diphenylmethyl-, 1-(4-Chlor- oder 4,4'-Dichlor-diphenylmethyl)-, 1-(4-Fluor- oder 4,4'-Difluor-diphenylmethyl)-, 1-(4-Chlor-4'-fluor-dipenylmethyl)-, 1-(Phenyl-4(3 oder 2)-pyridyl-methyl)-, 1-(4-Chlorphenyl-4-pyridyl-methyl)- und 1-(4-Methoxy- oder 4,4'-Dimethoxy-diphenylmethyl)-piperazin sowie 4-(Diphenyl-hydroxymethyl)-piperidin (Z=Zb: US—PS 2 804 422) und 4-Diphenylmethylen-piperidin (Z=Zc: FR—PS 2 042 313) in Betracht. Zur Herstellung der vorgenannten Piperazine kann man sich des in der DE—OS 27 14 437 (= GB—PS 1 579 365) beschriebenen Verfahrens bdienen, wonach 1-Formylpiperazin mit einer Verbindung der Formel V, beispielsweise Diphenyl-, 4-Chlorphenyl-phenyl-, Bis-(4-fluorphenyl)-, 4-Chlorphenyl-4-fluorphenyl-, Phenyl-4-pyridyl- und 4-Methoxyphenyl-phenyl-chlormethan, umgesetzt und anschließend die Formylgruppe unter alkalischen Bedingungen abgespalten wird.

Die Ausgangsverbindungen der Formel IV sind auf gleichem Wege durch Umsetzung von Verbindungen der Formel II mit 1-Formylpiperazin und nachfolgender Eliminierung der Formyl-Schutzgruppe durch alkalische Hydrolyse zugänglich.

Als geeignete Pyridinderivate der Formel VI kommen z.B. die 3-substituierten, 2,6-unsubstituierten, mono- oder dialkylierten Verbindungen mit 4-ständiger Amino-, Methylamino- oder Äthylaminogruppe und deren 1-Oxide in Frage, die man vorteilhaft aus den entsprechenden literaturbekannten 4-Halogen-Verbindungen der Formel VIII (z.B. DE—OS 29 00 504 und Patentanmeldung P 35 14 073) durch Umsetzung mit Ammoniak, Methyl- oder Äthylamin darstellen kann.

Die weiterhin als Ausgangsstoffe verwendeten cyclischen Amine der Formeln VII und IX sind größtenteils bekannt oder aber nach Literaturmethoden aus den voranstehend genannten Aminverbindungen der Formel III leicht herstellbar.

Die Umsetzung der jeweiligen Reaktionspartner II bis IX entsprechend den Verfahrensvarianten a), b), c) und d) wird zweckmäßig in einem gegenüber den Reaktionspartnern inerten Lösungs- oder Verteilungsmittel durchgeführt. Hierfür kommen z.B. Alkohole, wie Methanol, Äthanol, Isopropanol, n-Propanol, die verschiedenen Butanole, sowie Gemische derselben, oder auch deren Mischungen mit Äthern, wie Tetrahydrofuran und Dioxan, oder Kohlenwasserstoffen, wie Benzol, Toluol und Xylol, sowie aprotische Lösungsmittel, wie Pyridin, Dimethylformamid, Dimethylacetamid, Dimethylsulfoxyd und Hexamethylphosphorsäuretriamid in Frage.

Bei diesen Kondensationsreaktion arbeitet man vorteilhaft in Gegenwart einer mindestens zweifach molaren Menge des jeweils eingesetzten Amins; auch der Einsatz aquimolarer Mengen beider Reaktionspartner ist möglich doch empfiehlt sich dann die Zugabe eines säurebindenden Mittels, z.B. eines Alkali- oder Erdalkalihydroxids oder -carbonats oder auch einer organischen Base, wie Triäthylamin, in mindestens stöchiometrischer Menge. Die Reaktion wird im allgemeinen bei Temperaturen zwischen 0°C und dem Siedepunkt des jeweiligen Reaktionsmediums, vorzugsweise zwischen 20 und 100°C durchgeführt, wobei die Reaktionszeit bis zu mehreren Tagen betragen kann.

Die etwaige Umwandlung der erfindungsgemäßen 3-Nitroverbindungen der Formel I in die entsprechenden 3-Aminoverbindungen der Formel I gemäß Verfahrensvariante e) erfolgt in üblicher Weise, beispielsweise mit reduzierrend wirkenden Metallsalzen, wie Eisen(II)salzen, Eisen(II)ammonium-Komplexen, Sulfiten, Sulfiden, Dithioniten oder Titan(III)verbindungen, oder durch katalytische Reduktion mit Hydrazin oder bevorzugt Wasserstoff über Nickel-, Platin-, Ruthenium- oder vorzugsweise Palladium-Katalystoren. Die Reaktion wird — wie üblich- in einem gegenüber den Reaktionsteilnehmern inerten Lösungs-, oder Verteilungsmittel, vorzugsweise Wasser, einem Alkohol, wie Methanol, Äthanol oder Isopropanol, oder einem halogenierten Kohlenwasserstoff, wie Dichlormethan, Chloroform oder Tetrachlormethan, oder aber auch einem Gemisch aus diesen Lösungsmitteln vorgenommen, wobei Reaktionstemperaturen zwischen 0° und 100°C, vorzugsweise zwischen 20 und 80°C, angewendet werden. Die katalytische Hydrierung kann unter Anwendung höherer Drucke, biespielsweise in einer Parr-Apparatur unter einem Überdruck bis zu etwa 10 bar, oder vorzugsweise unter Atmosphärendruck in einer Schüttelapparatur erfolgen, im allgemeinen bei 0 bis 100°C, vorzugsweise bei Raumtemperatur.

Die Hydrolyse der erfindungsgemäßen 3-Carbonsäureester der Formel I zu den entsprechenden 3-Carbonsäuren der Formel I gemäß Verfahrensvariante f) wird bevorzugt unter alkalischen Bedingungen, gegebenenfalls bei erhöhten Temperaturen durchgeführt. Vorteilhaft arbeitet man in einem Lösungsmittel, wie Wasser, einem Äther, Keton oder niederen ein- oder zweiwertigen Alkohol, wie Diäthyläther, Diisopropyläther, Aceton, Methyläthylketon, Methylisobutylketon, Methanol, Äthanol, den verschiedenen Propanolen und Butanolen, dem Monomethyl- bzw. Monoäthyläther des Äthylenglykols, den beiden Propandiolen, vorzugsweise aber Äthylenglykol. Das alkalische Medium läßt sich z.B. durch Alkalihydroxyde oder -carbonate, insbesondere Natrium- oder Kaliumhydroxyd, einstellen.

Die Dehydratisierung von erfindungsgemäßen tertiären Alkoholen der Formel I mit dem Strukturelement der Formel Zb, worin $R^6$ eine Hydroxygruppe darstellt, zu den ungesättigten Verbindungen der Formel I mit dem Strukturmerkmal Zc nach Verfahrensvariante g) wird ebenfalls vorteilhaft in einem Lösungsmittel, wie verdünnten wäßrigen Mineralsäuren oder in mit Chlorwasserstoff gesättigten niederen ein- oder zweiwertigen Alkoholen, vorzugsweise Äthanol, bei Temperaturen von 0°C bis zum Siedepunkt des jeweiligen Reaktionsgemisches, vorzugsweise bei Raumtemperatur, vorgenommen.

Die erfindungsgemäßen Verbindungen der Formel I und ihre physiologisch verträglichen Salze können aufgrund ihrer pharmakologischen Eigenschaften als Arzneimittel, insbesondere als solche zur prophylaktischen und/oder kurativen Behandlung von atopischen Atemwegserkrankungen, wie allergischer Rhinitis, allergischem Asthma und anaphylaktischem Schock, sowie von allergischen Dermatitiden und allergischer Konjunktivitis Verwendung finden, wobei man sie entweder allein, z.B. in Form von Mikrokapseln, in Mischungen untereinander oder in Kombination mit geeigneten Hilfsstoffen, z.B. Trägerstoffen verabreicht.

Gegenstand der Erfindung sind somit auch Arzneimittel, die mindestens eine Verbindung der Formel I, gegebenenfalls in Form eines ihrer physiologisch verträglichen Salze, als Wirkstoff enthalten oder daraus bestehen, und eine echte Bereicherung, der Pharmazie darstellen.

Die erfindungsgemäßen Arzneimittel können oral, parenteral, inhalativ, rektal und gegebenenfalls auch epikutan verabreicht werden. Geeignete feste oder flüssige galenische Zubereitungsformen sind beispielsweise Granulate, Pulver, Tabletten, Dragees, (Mikro)Kapseln, Zäpfchen, Sirupe, Emulsionen, Suspensionen, Salben, Aerosole, Tropfen oder injizierbare Lösungen in Ampullenform, transdermale Applikationssysteme sowie Präparate mit protrahierter Wirkstoff-Freigabe, bei deren Herstellung gewöhnlich Hilfsmittel, wie Trägerstoffe, Spreng-, Binde-, Überzugs-, Quellungs, Gleit- oder Schmiermittel, Geschmacksstoffe, Süßungsmittel, Puffersubstanzen, Antioxidantien oder Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyäthylenglykole und Lösungsmittel, wie etwa steriles Wasser, Alkohole, Glycerin und andere mehrwertige Alkohole genannt.

Vorzugsweise werden die pharmazeutischen Präparate in Dosierungseinheiten hergestellt und verabreicht, wobei jede Einheit als aktiven Bestandteil eine bestimmte Dosis einer Verbindung gemäß Formel I, gegebenenfalls in Form eines ihrer physiologisch verträglichen Salze enthält. Bei festen Dosierungseinheiten, wie Tabletten, Suppositorien und Kapseln kann diese Dosis bis zu 100 mg, bevorzugt jedoch 5 bis 50 mg, und bei Injektionslösungen bis zu 20 mg, vorzugsweise 1 bis 15 mg betragen, wobei sich die Mengenangaben auf die Verbindung I als solche beziehen.

Für die inhalative Anwendung kommen Aerosole mit einem Wirkstoffgehalt von maximal 2%, vorzugsweise 0,5 bis 1%, in Frage.

Für die Behandlung eines erwachsenen Patienten sind — je nach Wirksamkeit der Verbindungen gemäß Formel I und ihrer Salze Tagesdosen bei oraler oder rektaler Verabreichung von 5 bis 100 mg

Wirkstoff, vorzugsweise 10 bis 60 mg, und bei intravenöser oder inhalativer Applikation 1 bis 40 mg, bevorzugt 3 bis 30 mg indiziert. Unter Umständen können jedoch auch höhere oder niedrigere Tagesdosen empfehlenswert sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Schließlich können die neuen Verbindungen gemäß Formel I sowie deren Salze bei der Herstellung der vorgenannten galenischen Zubereitungsformen auch zusammen mit anderen geeigneten Wirkstoffen, beispielsweise Corticosteroiden, Brochospasmolytica und Mucokinetika formuliert werden.

In den folgenden Beispielen wird unter "Vakuum" das der Wasserstrahlpumpe verstanden. DC bedeutet Dünnschicht-Chromatographie.

## Beispiele

Die Struktur der nachstehend beschriebenen Verbindungen wurde durch Elementaranalyse sowie IR- und $^1$H-NMR-Spektren bewiesen.

1) 2,6-Dimethyl-3-nitro-4-[(2-{4-diphenylmethyl-1-piperazinyl}-äthyl)-amino]-pyridin-1-oxid-trihydrochloriddihydrat:

11 g (0,045 Mol) 4-(2-Chloräthylamino-2,6-dimethyl-3-nitropyridin-1-oxid, 14,8 g (0,059 Mol) 1-Diphenylmethylpiperazin und 6 g (0,06 Mol) Triäthylamin wurden nach Verfahrensvariante a) in 200 ml Isopropanol 80 Stunden zum Sieden erhitzt. Das Lösungsmittelgemisch wurde in Vakuum abdestilliert und der Rückstand zwischen Dichlormethan und Wasser ausgeschüttelt. Die organische Phase wurde zur Trockne eigeengt, der Rückstand in Äthanol gelöst und diese Lösung mit äthanolischer HCl versetzt. Nach dem Abdestillieren des Äthanols wurde das Trihydrochlorid aus Isopropanol/Diisopropyläther (4:1; Vol) umkristallisiert:

$C_{26}H_{34}Cl_3N_5O_3 \times H_2O$ (MG: 606,95); Schmelzpunkt 219°C (Zers.).

Analyse:

| | | | | |
|---|---|---|---|---|
| Ber.: | C 51,45 | H 5,65 | Cl 17,52 | N 11,54 |
| Gef.: | C 51.23 | H 5,50 | Cl 17,79 | N 11,28 |

Die Verbindung konnte auch aus 2,6-Dimethyl-3-nitro- 4-[(2-{1-piperazinyl}-äthyl)-amino]-pyridin-1-oxid und Brom-diphenylmethan gemäß Verfahrensvariante b) hergestellt werden.

2) 4-[(2-{4-Benzyl-1-piperazinyl}-äthyl)-amino]-2,6-dimethyl-3-nitropyridin-trihydrochlorid:

13,3 g (0.05 Mol) 4-(2-Chloräthylamino)-2,6-dimethyl-3-nitropyridin, 8,56 g (0,075 Mol) 1-Formylpiperazin und 10 g (0.1 Mol) Triäthylamin wurden in 300 ml Isopropanol 10 Stunden zum Sieden erhitzt. Die Flüssigkeiten wurden im Vakuum abdestilliert und der verbleibende feste Rückstand zwischen Dichlormethan und Wasser ausgeschüttelt. Die Dichlormethanphase wurde eingeengt, der Rückstand einmal aus Isopropanol umkristallisiert und 2 Stunden in 6 N Salzsäure zum Sieden erhitzt. Mit 10%-iger Natronlauge wurde die Lösung anschließend auf pH 7 eingestellt und das Wasser abdestilliert. Der feste Ruckstand wurde an Kieselgel mit Dichlormethan/Methanol (8:2; Vol) gereinigt. Man erhielt 4,5 g (0.016 Mol) [31,8% der Theorie] an 2,6-Dimethyl-3-nitro-4-[(2-{1-piperazinyl}-äthyl]-amino]-pyridin (Schmelzpunkt des Trihydrochlorids 128—129°C), das mit 2,15 g (0,017 Mol) Benzylchlorid und 2 g (0.02 Mol) Triäthylamin nach Verfahrenvariante b) in 100 ml Isopropanol 6 Stunden zum Rückfluß erhitzt wurde. Die Lösungsmittel wurden abdestilliert und der ölige Rückstand mit Essigsäureäthylester ausgerührt, filtriert und das Filtrat zur Trockne eingeengt. Der Rückstand wurde in Äthanol aufgenommen, mit äthanolischer HCl versetzt und zur Trockne eingeengt. Der Eindampfrückstand wurde aus Isopropanol/Diisopropyläther (4:1; Vol) umkristallisiert. Ausbeute: 6,2 g (81% der Theorie).

$C_{20}H_{30}Cl_3N_5O_2$ (MG: 478,85); Schmelzpunkt 230°C.

Analyse:

| | | | | |
|---|---|---|---|---|
| Ber.: | C 50,17 | H 6,31 | Cl 22,21 | N 14,63 |
| Gef.: | C 50,02 | H 6,07 | Cl 21,93 | N 14,51 |

Die Verbindung ließ sich auch analog Verfahrensvariante d) aus 4-Chlor-2,6-dimethyl-3-nitropyridin und 1-(2-Aminoäthyl)-4-benzylpiperazin darstellen.

3) 2,6-Dimethyl-3-nitro-4-[(2-{4-diphenylmethyl-1-piperazinyl}-äthyl)-amino]-pyridin-trihydrochlorid:

Gemäß Verfahrensvariante c) wurden in einem 500 ml Rundkolben 10 g (0,08 Mol) 1-(2-Chloräthyl)-4-diphenylmethyl-piperazin, 13,4 g (0,08 Mol) 4-Amino-2,6-dimethyl-3-nitropyridin und 10 g (0,1 Mol) Triäthylamin in 300 ml Isopropanol gelöst und 10 Stunden zum Sieden erhitzt. Man kühlte auf Raumtemperatur ab und saugte den Niederschlag ab. Dieser wurde mit Essigsäureäthylester ausgerührt, filtriert und das Filtrat zur Trockne eingeengt. Der verbliebene Rückstand wurde in Äthanol aufgenommen und mit äthanolischer HCl versetzt. Im Vakuum wurde die Lösung zur Trockne eingeengt und der Rückstand aus Isopropanol/Diisopropyläther (4:1; Vol) umkristallisiert. Ausbeute: 18,7 g (39,4% der Theorie).

$C_{26}H_{34}Cl_3N_5O_2$ (MG: 554,95); Schmelzpunkt 248—250°C (Zers.).

Analyse:

| | | | | |
|---|---|---|---|---|
| Ber.: | C 56,27 | H 6,18 | Cl 19,17 | N 12,62 |
| Gef.: | C 56,03 | H 6,17 | Cl 19,05 | N 12,64 |

Die Verbindung wurde auch durch Reaktion von 4-(2-Chloräthylamino)-2,6-dimethyl-3-nitropyridin mit 1-Diphenylmethylpiperazin gemäß Verfahrensvariante a) in Ausbeuten bis zu 70% erhalten.

4) 3-Amino-2,6-dimethyl-4-[(2-{4-diphenylmethyl-1-piperazinyl}-äthyl)-amino]-pyridin-trihydrochlorid-dihydrat:

13,5 g (0,03 Mol) Base der Nitroverbindung aus Beispiel 3 wurden gemäß Verfahrensvariante e) in einer Mischung aus 100 ml Methanol und 70 ml Dichlormethan gelöst und über 1 g Palladium (10%) auf Aktivkohle in einer Schüttelapparatur bei Raumtemperatur mit Wasserstoff hydriert. Nachdem keine Nitroverbindung mehr vorhanden war (DC-Kontrolle), wurde der Katalysator abfiltriert und das Lösungsmittelgemisch abdestilliert. Der amorphe Rückstand wurde zwischen Dichlormethan und Wasser, das mit 1 N Salzsäure auf pH 6 gebracht war, ausgeschüttelt. Die eingeengte organische Phase wurde in Äthanol gelöst, mit äthanolischer HCl versetzt, der entstandene Niederschlag abgesaugt und aus Isopropanol umkristalliert, Ausbeute: 7,2 g (42,8% der Theorie).

$C_{26}H_{36}Cl_3N_5 \times 2\ H_2O$ (MG: 564,97); Schmelzpunkt 210—212°C

Analyse:

| | | | | |
|---|---|---|---|---|
| Ber.: | C 55,27 | H 7,14 | Cl 18,83 | N 12,40 |
| Gef.: | C 54,98 | H 7,02 | Cl 18,90 | N 12,41 |

5) 2,6-Dimethyl-4-[(2-{4-diphenylmethyl-1-piperazinyl}-äthyl)-amino]-pyridin-3-carbonsäure-trihydrochlorid-dihydrat:

Eine Mischung aus 8 g (0,017 Mol) 2,6-Dimethyl-4-[(2-{4-diphenylmethyl-1-piperazinyl}-äthyl)-amino]-pyridin-3-carbonsäureäthylester (Beispiel 11) und 1,4 g (0,025 Mol) Kaliumhydroxyd wurde nach Verfahrensvariante f) 2 Stunden in Äthylenglykol auf 170°C erhitzt. Nach dem Abkühlen auf Raumtemperatur wurde die Lösung mit 4 N Salzsäure auf pH 4 gestellt. Der dabei entstandene Niederschlag wurde abgesaugt, mit Wasser gewaschen, getrocknet und mit äthanolischer HCl ins Hydrochlorid übergeführt. Durch Zugabe von Diisopropyläther fiel das Trihydrochlorid-dihydrat aus und wurde aus Isopropanol/Diisopropyläther (4:1; Vol.) umkristallisiert. Ausbeute: 6,5 g (65% der Theorie).

$C_{27}H_{35}CL_3N_4O_2 \times 2\ H_2O$ (MG: 589,96); Schmelzpunkt 248—249°C (Zers.).

Analyse:

| | | | | |
|---|---|---|---|---|
| Ber.: | C 54,97 | H 6,66 | Cl 18,03 | N 9,50 |
| Gef.: | C 54.72 | H 6,92 | Cl 17,90 | N 9,57 |

Die Verbindung war auch durch Umsetzung von 4-(2-Chloräthylamino)-2,6-dimethyl-pyridin-3-carbonsäure und 1-Diphenylmethylpiperazin nach Verfahrensvariante a) zugänglich.

6) 2,6-Dimethyl-3-nitro-4-[(2-{4-diphenylmethylen-1-piperidyl}-äthyl)-amino]-pyridin-dihydrochlorid-dihydrat:

10 g (0,02 Mol) 4-[(2-{4-(Hydroxy-diphenyl-methyl)-1-piperidyl}-äthyl)-amino]-2,6-dimethyl-3-nitropyridin-dihydrochlorid (Beispiel 17) wurden gemäß Verfahrensvariante g) in 100 ml Äthanol gelöst und mit HCl-Gas unter Eiskühlung gesättigt. Diese Lösung wurde über Nacht stehen gelassen und eine Stunde zum Rückfluß erhitzt. Das Lösungsmittel wurde abdestilliert und der verbleibende Rückstand mehrmals aus Isopropanol/Diisopropyläther (8:2; Vol.) umkristallisiert. Ausbeute: 3,5 g (31% der Theorie).

$C_{27}H_{32}Cl_2N_4O_2 \times 2\ H_2O$ (MG: 551,49); Schmelzpunkt 225—226°C.

Analyse:

| | | | |
|---|---|---|---|
| Ber.: | C 62,91 | H 6,26 | N 10,87 |
| Gef.: | C 63.07 | H 6,24 | N 11,12 |

Die Verbindung ist auch durch Umsetzung von 4-(2-Chloräthylamino)-2,6-dimethyl-3-nitropyridin mit 4-Diphenylmethylenpiperidin gemäß Verfahrensvariante a) zugänglich.

Die vorgenannten und auf analoge Weise hergestellten Verbindungen sind in nachstehender Tabelle 1 zusammengefaßt.

TABELLE 1
Verbindungen gemäß Formel I (siehe Anspruch 1)

| Beispiel | $R^1$ | $R^2$ | $R^3$ | A | Z | n | X | isoliert als | Schmelzpunkt °C |
|---|---|---|---|---|---|---|---|---|---|
| 1 | $CH_3$ | $CH_3$ | H | $—C_2H_4—$ | 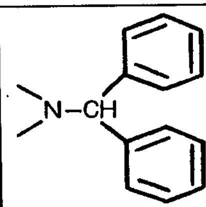 | 1 | $NO_2$ | 3 HCl<br>2 $H_2O$ | 219 (Zers.) |
| 2 | $CH_3$ | $CH_3$ | H | $—C_2H_4—$ | 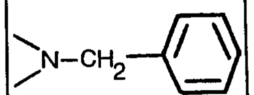 | 0 | $NO_2$ | 3 HCl | 230 |
| 3 | $CH_3$ | $CH_3$ | H | $—C_2H_4—$ | 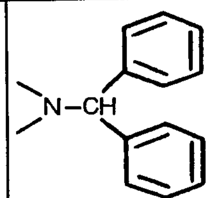 | 0 | $NO_2$ | 3 HCl | 248—250 (Zers.) |
| 4 | $CH_3$ | $CH_3$ | H | $—C_2H_4—$ | 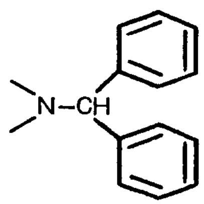 | 0 | $NH_2$ | 3 HCl<br>2 $H_2O$ | 210—212 |
| 5 | $CH_3$ | $CH_3$ | H | $—C_2H_4—$ | 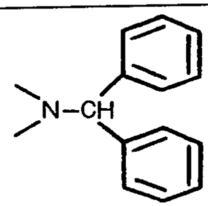 | 0 | $CO_2H$ | 3 HCl<br>2 $H_2O$ | 248—249 (Zers.) |

TABELLE 1 (Fortsetzung)

| Beispiel | $R^1$ | $R^2$ | $R^3$ | A | Z | n | X | isoliert als | Schmelzpunkt °C |
|---|---|---|---|---|---|---|---|---|---|
| 6 | $CH_3$ | $CH_3$ | H | $—C_2H_4—$ | 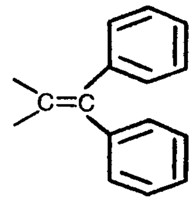 | 0 | $NO_2$ | 2 HCl<br>2 $H_2O$ | 225—226 |
| 7 | $CH_3$ | $CH_3$ | H | $—C_3H_6—$ | 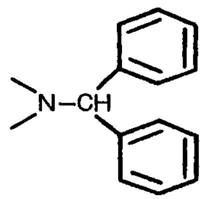 | 0 | $NO_2$ | 3 HCl | 203—204 |
| 8 | $CH_3$ | $CH_3$ | H | $—C_2H_4—$ | 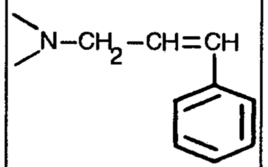 | 0 | $NO_2$ | 3 HCl<br>2,5 $H_2O$ | 225—226 |
| 9 | $CH_3$ | $CH_3$ | H | $—C_2H_4—$ | 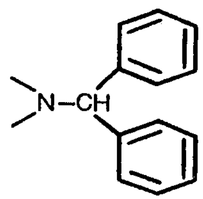 | 0 | CN | 3 HCl | 225—227 |
| 10 | $CH_3$ | $CH_3$ | H | $—C_2H_4—$ | 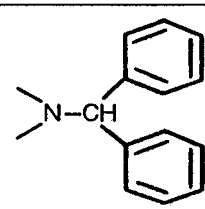 | 1 | CN | 3 HCl<br>1 $H_2O$ | 219 |

TABELLE 1 (Fortsetzung)

| Beispiel | $R^1$ | $R^2$ | $R^3$ | A | Z | n | X | isoliert als | Schmelzpunkt °C |
|---|---|---|---|---|---|---|---|---|---|
| 11 | $CH_3$ | $CH_3$ | H | $-C_2H_4-$ | 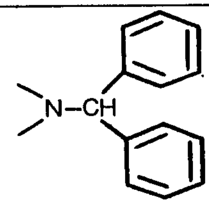 | 0 | $CO_2C_2H_5$ | 2 HCl<br>2 $H_2O$ | 220 |
| 12 | $C_3H_7$ | $C_3H_7$ | H | $-C_2H_4-$ | 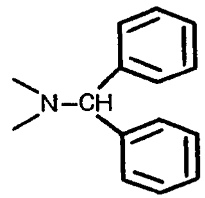 | 0 | $NO_2$ | 2 HCl | 222 |
| 13 | $CH_3$ | $CH_3$ | $CH_3$ | $-C_2H_4-$ | 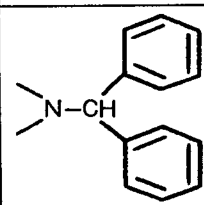 | 0 | $NO_2$ | 3 HCl<br>1 $H_2O$ | 214 |
| 14 | $CH_3$ | $CH_3$ | H | $-C_2H_4-$ | 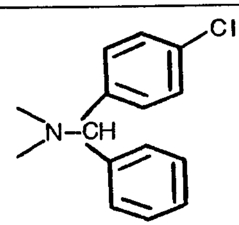 | 0 | $NO_2$ | 3 HCl<br>2 $H_2O$ | 210 (Zers.) |
| 15 | $CH_3$ | $CH_3$ | H | $-C_2H_4-$ | 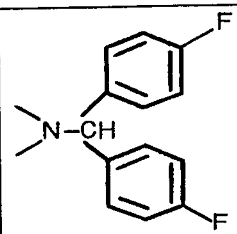 | 0 | $NO_2$ | 3 HCl<br>3 $H_2O$ | 197 |

TABELLE 1 (Fortsetzung)

| Beispiel | $R^1$ | $R^2$ | $R^3$ | A | Z | n | X | isoliert als | Schmelzpunkt °C |
|---|---|---|---|---|---|---|---|---|---|
| 16 | $CH_3$ | $CH_3$ | H | $-C_2H_4-$ | 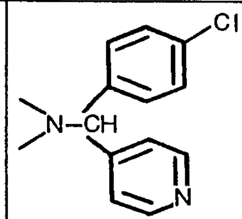 | 0 | $NO_2$ | 4 HCl | 105 |
| 17 | $CH_3$ | $CH_3$ | H | $-C_2H_4-$ | 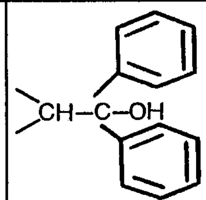 | 0 | $NO_2$ | 2 HCl | 217—219 |
| 18 | $CH_3$ | $CH_3$ | H | $-C_2H_4-$ | 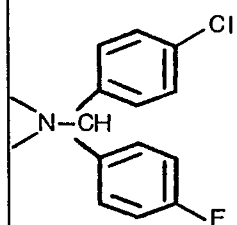 | 0 | $NO_2$ | 3 HCl<br>2 $H_2O$ | 205 |
| 19 | $CH_3$ | $CH_3$ | H | $-C_2H_4-$ | 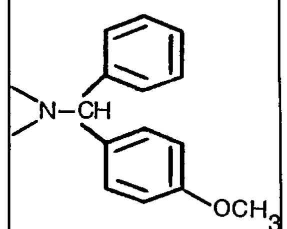 | 0 | $NO_2$ | 3 HCl<br>2 $H_2O$ | 155—158 |

TABELLE 1 (Fortsetzung)

| Beispiel | $R^1$ | $R^2$ | $R^3$ | A | Z | n | X | isoliert als | Schmelzpunkt °C |
|---|---|---|---|---|---|---|---|---|---|
| 20 | $CH_3$ | $CH_3$ | H | $-C_2H_4-$ | 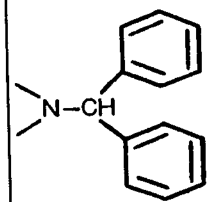 | 0 | H | 3 HCl | 195—196 |
| 21 | H | H | H | $-C_2H_4-$ | 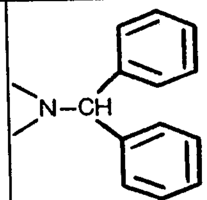 | 0 | $NO_2$ | 3 HCl<br>1 $H_2O$ | 198—199 |
| 22 | H | $CH_3$ | H | $-C_2H_4-$ | 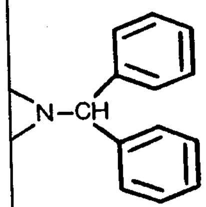 | 0 | $NO_2$ | 3 HCl | 237 (Zers.) |
| 23 | $CH_3$ | $CH_3$ | H | $-C_2H_4-$ | 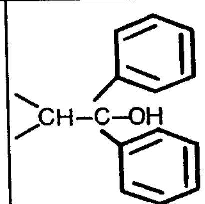 | 0 | $CO_2CH_3$ | 3 HCl | 127—228 |

TABELLE 1 (Fortsetzung)

| Beispiel | $R^1$ | $R^2$ | $R^3$ | A | Z | n | X | isoliert als | Schmelzpunkt °C |
|---|---|---|---|---|---|---|---|---|---|
| 24 | $CH_3$ | $CH_3$ | H | $—C_2H_4—$ | 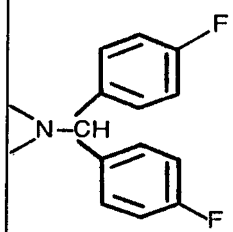 | 0 | $CO_2H$ | 3 HCl<br>2 $H_2O$ | 161 (Zers.) |
| 25 | $CH_3$ | $CH_3$ | H | $—C_2H_4—$ | 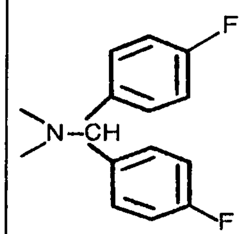 | 0 | $NH_2$ | 3 HCl<br>2 $H_2O$ | 190—197 |

Pharmakologische Prüfung und Ergebnisse

1. Antagonistische Wirkung gegenüber den Allergiemediatoren (am narkotisierten Meerschweinchen)

Die Prüfung der erfindungsgemäßen Verbindungen auf ihre Hemmwirkung gegenüber den spasmogenen Aminen Histamin und Bradykinin erfolgte mit der von H. Konzett und R. Rössler beschriebenen Versuchsanordnung (Arch. Exp. Path. u. Pharmak. *195* (1940), Seite 71) im Vergleich mit dem wichtigsten Vertreter der zum Stand der Tecknik gehörenden, bronchospasmolytisch wirksamen Pyridin-Reihe gemäß DE—OS 29 00 504, dem 4-(2,6-Dimethyl-3-nitro-4-pyridyl)-thiomorpholinhydrochlorid (Beispiel 1).

Bei dieser Methode wird die Hemmung experimentell durch intravenöse (i.v.) Gabe von Histamin oder Bradykinin ausgelöster Brochospasmen an Meerschweinchen männlichen Geschlechts in Urethan-Narkose (1,25 g/kg i.p.) untersucht. Die Prüfsubstanzen wurden in wäßriger Lösung intravenös in einem Injektionsvolumen von 1 ml/kg verabreicht.

Als Beurteilungskriterium für die Hemmwirkung dienten die $ED_{50}$-Werte bzw. -Bereiche, worunter jene Dosen in mg/kg verstanden werden, mit denen der experimentell erzeugte Spasmus auf die Hälfte gegenüber jenem unbehandelter Kontrolltiere herabgesetzt werden konnte.

2. Hemmung der Freisetzung von Allergiemediatoren (Mastzellprotektiver Effekt)

a) *Passive cutane Anaphylaxie (PCA) an der Ratte (PCA-Test)*

Die passive cutane Anaphylaxie stellt eine IgE-vermittelte Allergie vom Sofort-Typ (Typ-I) dar. In diesem Modell reagieren an Mastzellen und basophilen Granulocyten gebundene Antikörper mit einem intravenös applizierten Antigen unter exzessiver Freisetzung der Allergiemediatoren. Das dabei freiwerdende Histamin läßt sich durch gleichzeitige Gabe von Evansblau an den rasierten Flanken der Ratten sichtbar machen.

Durch photometrische Auswertung der Farbintensität wurde der Mastzell-protektive Effekt der erfindungsgemäßen Verbindung nach oraler Verabreichung anhand der prozentualen Hemmung der Mediatorfreisetzung im Vergleich zu unbehandelten Kontrolltieren bestimmt. Auch in diese Untersuchung wurde die voranstehend genannte Verbindung des Beispiels 1 aus der DE—OS 29 00 504 miteinbezogen, wobei sie sich als unwirksam und daher ungeeignet für die Therapie allergischer Erkrankungen erwies.

b) *Durch Clacium-Ionophor A 23 187 induzierte Histamin-freisetzung aus isolierten Rattenmastzellen*

Mit Hilfe bestimmter Agentien, beispielsweise des Calcium-Ionophors A 23 187 und basischer Peptide, kann man Mastzellen — ähnlich wie bei der IgE-vermittelten Immunreaktion — zur exzessiven Freisetzung von Allergiemediatoren anregen, von denen sich das ins Medium übergetretene Histamin relativ einfach quantitativ erfassen läßt.

Über die Hemmung dieser artifiziell induzierten Histamin-freisetzung aus peritonealen Rattenmastzellen wurde die Mastzell-protektive Wirkung der erfindungsgemäßen Verbindungen bestimmt. Hierzu wurden $10^4$ Zellen in dem Gewebekulturmedium Clicks/RPMI (50:50, Vol.; Fa. Serva, Heidelberg) suspendiert, 15 Minuten lang mit der Testsubstanz inkubiert und anschließend mit dem Calcium-Ionophor A 23 187 in einer Konzentration von $10^{-6}$ g/ml behandelt. Die Bestimmung des freigesetzten Histamins erfolgte durch Hochdruckflüssigkeitschromatographie (HPLC). Als Maß für die Hemmwirkung dienten die $IC_{50}$-Werte, die jene molaren Konzentrationen (mol/l) darstellen, mit denen die induzierte Histaminfreisetzung um die Hälfte gegenüber jener nicht mit einem Präparat vorbehandelter Zellen herabgesetzt wurde. Hierbei gelten $IC_{50}$-Werte oberhalb $10^{-5}$ mol/l aus therapeutischer Sicht als irrelevant. Demzufolge erwies sich das Vergleichspräparat gemäß Beispiel 1 aus der DE—OS 29 00 504 auch in diesem Test als inaktiv.

3. Akute Toxizität

Die Bestimmung der $LD_{50}$-Werte erfolgte standardgemäß über die innerhalb von 7 Tagen bei NMRI-Mäusen (NMRI = Naval Medical Research Institute) nach einmaliger intraperitonealer (i.p.) Gabe auftretende Mortalität.

Die Ergebnisse dieser Untersuchungen sind in den nachfolgenden Tabellen 2 und 3 zusammengestellt.

TABELLE 2
Antagonistische Wirkung gegenüber den Allergiemediatoren und akute Toxizität

| Verbindung des Beispiels | Hemmwirkung ($ED_{50}$ in mg/kg i.v.) gegenüber Histamin | Bradykinin | Akute Toxizität $LD_{50}$ (Maus i.p.) in mg/kg |
|---|---|---|---|
| 1 | 0,3—1 | 1—3 | >150 |
| 3 | 0,3 | 0,15 | 300—600 |
| 5 | 1—3 | 3 | 300—600 |
| 6 | 1—3 | 3—6 | 600—1200 |
| 7 | 3 | 3—10 | 150—300 |
| 8 | 3 | 3—10 | 75—150 |
| 9 | 0,3—1 | 3—10 | 300—600 |
| 10 | 0,1—0,3 | 1—3 | 75—150 |
| 13 | 1—3 | 10 | 150—300 |
| 14 | 1—3 | 3—10 | 150—300 |
| 15 | 1—3 | 3—6 | 300—600 |
| 16 | 1 | 1 | 300—600 |
| 17 | 0,3 | 3—10 | 75—150 |
| 21 | 0,02 | 0,03 | 150—300 |
| Verbindung des Beispiels 1 aus DE—OS 29 00 504 | 1,05 | 3,16 | 150—300 |

TABELLE 3
Hemmung der Freisetzung von Allergiemediatoren (Mastzell-protektive Wirkung)

| Verbindung des Beispiels | PCA—Test Dosis in mg/kg p.o. | PCA—Test Hemmung der PCA in % | Hemmwirkung im Ca-Ionophor-Test $IC_{50}$ in mol/l |
|---|---|---|---|
| 1 | 50 | 91 | $10^{-7}$ |
| 2 | 50 | 67 | $10^{-5}$ |
| 3 | 50<br>25<br>12,5 | 85<br>61<br>26 | $10^{-8}$ |
| 4 | 50 | 73 | $5 \cdot 10^{-8}$ |
| 5 | 50 | 91 | $10^{-7}$ |
| 6 | 50 | 91 | n.g. *) |
| 7 | 50 | 55 | $10^{-8}$ |
| 8 | 50 | 60 | $10^{-7}$ |
| 9 | 50 | 64 | $10^{-8}$ |
| 10 | 50 | 91 | $< 10^{-8}$ |
| 12 | 50 | 61 | n.g. *) |
| 13 | 50 | 68 | $< 10^{-8}$ |
| 14 | 50 | 55 | $10^{-6}$ |
| 15 | 50 | 59 | $10^{-7}$ |
| 16 | 50<br>25 | 75<br>46 | $10^{-7}$ |
| 17 | 50 | 91 | $< 10^{-8}$ |
| Verbindung des Beispiels 1 aus DE—OS 29 00 504 | 50<br>25<br>12,5 | 13<br>0<br>0 | $> 10^{-4}$ |

*) n.g. bedeutet nicht getestet

Bei der Prüfung im Konzett-Rössler-Modell fiel auf, daß die Verbindungen der Beispiele 4 und 5 zusätzlich eine starke anticholinerge Wirkung besitzen, so daß sich diese Präparate auch zur Behandlung von Bronchospasmen nichtallergischer Genese eignen.

Auch in weiteren Spezialversuchen konnte eindrucksvoll gezeigt werden, daß die erfindungsgemäßen Verbindungen das eingangs erwähnte optimale Wirkungsprofil besitzen und daher allen zum Stand der Technik genannten Präparaten eindeutig überlegen sind:

Auf den am präsensibilisierten Meerschweinchen mit Ovalbumin (1 mg/kg i.v.) ausgelösten allergischen Bronchospasmus üben die Verbindungen der Formel I und deren Salze ebenfalls eine starke Hemmwirkung aus. So liegt beispielsweise der $ED_{50}$-Wert für die Verbindung des Beispiels 3 zwischen 0,1 und 0,3 mg/kg i.v.

Die Wirksamkeit der erfindungsgemäßen Verbindungen bei inhalativer Anwendung wurde durch Hemmung des mit Histamin induzierten allergischen Asthmas am wachen Meerschweinchen nachgewiesen. So führte die Inhalation etwa der Verbindung des Beispiels 3 in Form eines nur 0,3 %-igen Aerosols zu einer weitgehenden Unterdrückung der experimentell ausgelösten Asthmaanfälle.

Die Verbindungen der Formel I und deren Salze zeigen weiterhin eine ausgeprägte Hemmwirkung gegenüber dem plättchenaktivierenden Faktor (PAF) und den Leukotrienen, die ebenfalls im Verlaufe der anaphylaktischen Sofortreaktion als spasmogene Mediatoren aus den Mastzellen freigesetzt werden. Der an narkotisierten Ratten durch PAF-Gabe hervorgerufene Brochospasmus ließ sich beispielsweise durch die Verbindung des Beispiels 3 bei intravenöser Verabreichung mit einer $ED_{50}$ von 1—3 mg/kg antagonisieren. Die zum Stand der Technik genannten Präparate, wie Oxatomid, Ketotifen und Terfenadin, sind in diesem Test wirkungslos. Die durch den Calcium-Ionophor A 23 187 an isolierten Lungenstreifen und der isolierten Trachea des Meerschweinchens induzierten Kontraktionen wurden etwa von der Verbindung des Beispiels 3 mit einer $IC_{50}$ von 0,1—0,3 µg/ml gehemmt. Da der Calcium-Ionophor A 23 187 die 5-Lipoxygenase stimuliert und damit die Bildung der spasmogenen Leukotriene forciert, kann das Ergebnis dieses Versuches dahingehend interpretiert werden, daß die erfindungsgemäßen Verbindungen potente Leukotriene-Hemmer darstellen.

Der Nachweis einer unmittelbaren antagonistischen Wirkung gegenüber den Leukotrienen gelang schließlich mit einer Testanordnung, bei der an der isolierten Trachea des Meerschweinchens mit dem Leukotrien $D_4$ (10 pg/ml) ein langsam ansteigender und langanhaltender Spasmus erzeugt wurde, der sich beispielsweise mit der Verbindung des Beispiels 3 im Konzentrationsbereich von 3—6 µm/ml um 50% hemmen ließ.

In gründlichen psychopharmakologischen Untersuchungen konnte sichergestellt werden, daß die erfindungsgemäßen Verbindungen keine sedative Nebenwirkung aufweisen. Als Ausdruck einer zentralen Sedation vermindert beispielsweise Ketotifen die Spontanmotilität der Maus nach oraler Verabreichung von nur 1 mg/kg bereits signifikant, während die Verbindungen der Formel I diesen Parameter selbst in hohen Dosen bis zu 50 mg/kg per os nicht beeinflussen.

Plethysmographische Untersuchungen an wachen Meerschweinchen mit experimentell durch Inhalation von 0,3 %-igem Histamin-Aerosol erzeugter Atemwegsobstruktion haben gezeigt, daß die erfindungsgemäßen Verbindungen ihre Hemmwirkung auch nach oraler Applikation sehr schnell entfalten. So erreichte beispielsweise die Verbindung des Beispiels 3 mit einer $ED_{50}$ von nur 3,15 mg/kg p.o. ihr Wirkungsmaximum bereits 30 Minuten post applicationem, während etwa für das Präparat Oxatomid die entsprechenden Werte zu 25 mg/kg p.o. und 90 Minuten ermittelt wurden.

Schließlich beeinflußte die Verbindung des Beispiels 3 die normale Gewichtsentwicklung von Ratten in einem subchronischen 14 Tage-Versuch selbst in hohen oralen Dosen nicht. Daraus geht hervor, daß die Verbindungen der Formel I im Gegensatz zu vielen anderen Antihistaminka keine appetitanregende Wirkung besitzen.

**Patentansprüche für die Vertragsstaaten: BE CH DE FR GB IT LI LU NL SE**

1. Pyridinverbindungen der Formel I,

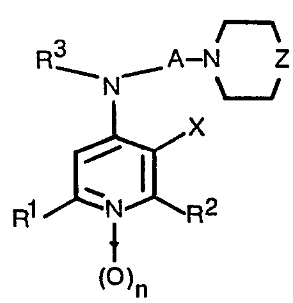

(I)

in der
$R^1$ und $R^2$ unabhängig voneinander für Wasserstoff oder Alkyl mit 1 bis 4 C-Atomen stehen,
$R^3$ Wasserstoff oder Alkyl mit 1 oder 2 C-Atomen und
A geradkettiges oder verzweigtes Alkylen mit 2 bis 4 C-Atomen bedeuten,
n den Wert 0 oder 1 hat,
Z eine Gruppe der Formel Za, Zb oder Zc

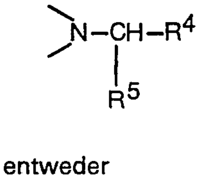 (Za) 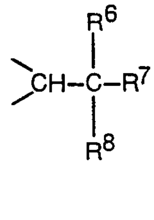 (Zb) 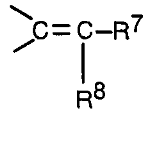 (Zc)

darstellt, wobei entweder
$R^4$ Wasserstoff und
$R^5$ Phenyl oder Cinnamyl oder

$R^4$, $R^5$, $R^7$ und $R^8$ unabhängig voneinander jeweils Pyridyl oder Phenyl, wobei das Phenyl gegebenenfalls bis zu zwei gleiche oder verschiedene Substituenten aus der Gruppe Halogen und Alkoxy mit bis zu 2 C-Atomen trägt,

$R^6$ Wasserstoff oder Hydroxy und

X Wasserstoff, eine Cyan-, Amino- oder Nitrogruppe oder den Rest —CO—$R^9$ bedeutet, in dem $R^9$ für Hydroxy oder Alkoxy mit 1 bis 4 C-Atomen steht,

und die physiologisch verträglichen Salze dieser Verbindungen.

2. Verbindungen und deren Salze nach Anspruch 1, gekennzeichnet durch wenigstens eines der Merkmale, daß die Reste $R^1$ und $R^2$ zusammen nicht mehr als 6 C-Atome enthalten, daß das Halogen, sofern vorhanden, an den Phenylringen Fluor, Chlor oder Brom darstellt und daß die Alkylenbrücke A Äthylen bedeutet.

3. Verbindungen und deren Salze nach Anspruch 1 oder 2, dadurch gekennzeichnet, daß in Formel I $R^1$ und $R^2$ zusammen nicht mehr als 2 C-Atome enthalten.

4. Verbindungen und deren Salze nach einem oder mehreren der Ansprüche 1 bis 3, dadurch gekennzeichnet, daß in Formel I $R^1$ und $R^2$ jeweils Methyl, $R^3$ Wasserstoff, A Äthylen, Z die Gruppe der Formel Za oder Zb und X die Cyan- oder Nitrogruppe bedeuten.

5. Verbindungen und deren Salze nach einem oder mehreren der Ansprüche 1 bis 4, dadurch gekennzeichnet, daß in Formel I Z die Gruppe der Formal Za darstellt, in der $R^4$ und $R^5$ die Bedeutung von Phenyl haben, das gegenbenenfalls mit bis zu zwei gleichen oder verschiedenen Halogenatomen substituiert ist.

6. Verbindungen und deren Salze nach Anspruch 5, dadurch gekennzeichnet, daß es sich um 3-Nitro- oder 3-Cyan-2,6-dimethyl-4-[(2-{4-diphenylmethyl-1-piperazinyl}-äthyl)-amino]-pyridin und deren 1-Oxide handelt.

7. Verfahren zur Herstellung von Pyridinverbindungen der Formel I gemäß einem oder mehreren der Ansprüche 1 bis 6 und ihren physiologisch verträglichen Salzen, dadurch gekennzeichnet, daß man

a) eine Verbindung der Formel II

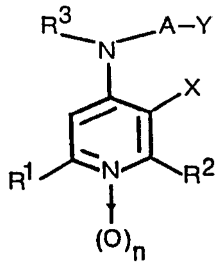

(II)

mit einem cyclischen Amin der Formel III

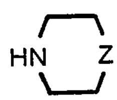

(III)

umsetzt, wobei $R^1$ bis $R^3$, n, X, A und Z mit $R^4$ bis $R^8$ die in einem oder mehreren Ansprüche 1 bis 6 genannten Bedeutungen haben und Y für eine Abgangsgruppe steht, oder

b) eine Verbindung der Formel IV

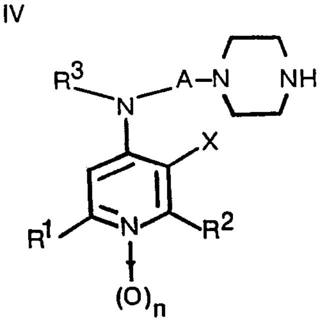

(IV)

mit einer Verbindung der Formel V

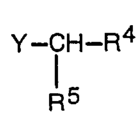

zu ener Verbindung der Formel I mit dem Strukturelement Za umsetzt, wobei $R^1$ bis $R^3$, n, X und A die in einem oder mehreren der Ansprüche 1 bis 6 definierten und Y die voranstehend genannten Bedeutungen haben, oder

c) eine Verbindung der Formel VI

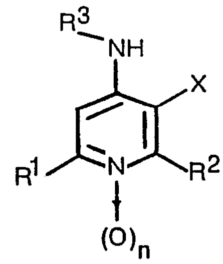

(VI)

mit einem cyclischen Amin der Formel VII

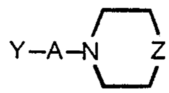

(VII)

umsetzt, wobei $R^1$ bis $R^3$, n, X, A und Z mit $R^4$ bis $R^8$ die in einem oder mehreren der Ansprüche 1 bis 6 und Y die voranstehend definierten Bedeutungen haben, oder

d) eine Verbindung der Formel VIII

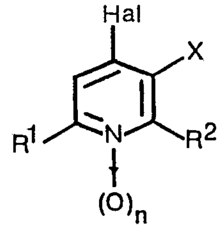

(VIII)

mit einem Amin der Formel IX

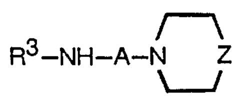

(IX)

umsetzt, wobei $R^1$ bis $R^3$, n, X, A und Z mit $R^4$ bis $R^8$ die in einem oder mehreren der Ansprüche 1 bis 6 genannten Bedeutungen haben und Hal ein Halogenatom darstellt,
daß man die Umsetzung gemäß den Verfahrensvarianten a), b), c) und d) in einem gegunüber den Reaktionspartnern inerten Lösungs- oder Verteilungsmittel in Gegenwart eines Amin-Überschusses oder unter Zusatz eines anderen säurebindenden Mittels bei Temperaturen zwischen 0°C und dem Siedepunkt des jeweiligen Reaktionsmediums durchführt und das gemäß diesen Verfahrensvarianten erhaltene Produkt isoliert, wobei man

e) eine Verbindung der Formel I, in der X für die Aminogruppe steht, auch herstellen kann, indem man eine gemäß a), b), c) oder d) erhaltene 3-Nitroverbindung der Formel I mit reduzierend wirkenden Metallsalzen oder durch katalytische Reduktion mit Hydrazin oder Wasserstoff in einem gegenüber den Reaktionsteilnehmern inerten Lösungs- oder Verteilungsmittel bei Temperaturen zwischen 0°C und 100°C bei Atmosphären- oder erhöhtem Druck reduziert, oder

f) eine Verbindung der Formel I, in der X die Carboxylgruppe bedeutet, auch herstellen kann, indem man einen gemäß a), b), c) oder d) erhaltenen 3-Carbonsäureester der Formel I, bevorzugt unter alkalischen Bedingungen, gegebenenfalls bei erhöhter Temperatur, hydrolyziert oder

g) eine Verbindung der Formel I mit dem Strukturmerkmal Zc auch herstellen kann, indem man eine gemäß a), b), c) oder d) erhaltene Verbindung der Formel I mit dem Strukturelement Zb, worin $R^6$ die Bedeutung von Hydroxy hat, in saurem Medium bei Temperaturen zwischen 0°C und dem Siedepunkt des jeweiligen Reaktionsgemisches, dehydratisiert,
wobei man die Verbindungen der Formel I entweder in freier Form isoliert oder sie mit geeigneten Säuren, oder für den Fall, daß X eine Carboxylgruppe darstellt, auch mit geeigneten Basen in physiologisch verträgliche Salze umwandelt.

8. Verfahren nach Anspruch 7, gekennzeichnet durch wenigstens eines der Merkmale, daß man die Umsetzung der Pyridinderivate gemäß Formeln II, IV, VI und VIII mit den Verbindungen der Formeln III, V, VII bzw. IX bei Temperaturen zwischen 20 und 100°C durchführt, daß man bei der Reduktion der 3-Nitroverbindugen der Formel I zu den entsprechenden 3-Aminoverbindungen mit katalytisch erregtem Wasserstoff bei Raumtemperatur und bei Atmosphärendruck arbeitet, daß man die alkalischen Bedingungen für die Hydrolyse der 3-Carbonsäureester der Formel I zu den korrespondierenden Carbonsäuren mit Alkalihydroxiden oder -carbonaten einstellt und daß man die Dehydratisierung der tertiären Alkohole der Formel I mit alkoholischer HCl bei Raumtemperatur vornimmt.

9. Arzneimittel, gekennzeichnet durch einen Gehalt an — oder bestehend aus — mindestens einer Verbindung der Formel I und/oder einem ihrer physiologisch verträglichen Salze nach einem oder

mehreren der Ansprüche 1 bis 6 oder mindestens einer nach dem Verfahren gemäß den Ansprüchen 7 oder 8 hergestellten Verbindung.

10. Arzneimittel nach Anspruch 9, dadurch gekennzeichnet, daß sie zur Vorbeugung und/oder Behandlung von allergisch konditionierten Erkrankungen bestimmt sind.

11. Arzneimittel nach Anspruch 9 oder 10, dadurch gekennzeichnet, daß sie zur Vorbeugung und/oder Behandlung von allergischer Rhinitis, allergischem Asthma, anaphylaktischem Schock, allergischen Dermatitiden und allergischer Konjunktivitis, bestimmt sind.

12. Dosierungseinheit eines Arzneimittels nach Anspruch 9, 10 oder 11, dadurch gekennzeichnet, daß sie eine wirksame Menge wenigstens einer Verbindung der Formel I enthält, wobei diese Dosiereinheit zweckmäßig entweder als feste Dosierungseinheit vorliegt, die bis zu 100 mg der Verbindung I enthält, oder als Injektionslösung, die bis zu 20 mg der Verbindung I enthält, wobei die Verbindung I als solche oder in Form eines physiologisch verträglichen Salzes vorliegt.

13. Dosierungseinheit nach Anspruch 12, dadurch gekennzeichnet, daß sie als feste Dosierungseinheit 5—50 mg der Verbindung I und als Injektionslösung 1—15 mg der Verbindung I enthält, wobei die Verbindung I als solche oder in Form eines physiologisch verträglichen Salzes vorliegt.

14. Verwendung von Pyridinverbindungen der Formel I und/oder deren physiologisch verträglichen Salzen gemäß einem oder mehreren der Ansprüche 1—6 zur Herstellung von Arzneimitteln, die zur Vorbeugung und/oder Behandlung von allergisch konditionierten Erkrankungen bestimmt sind.

**Patentansprüche für die Vertragsstaaten: GR ES AT**

1. Verfahren zur Herstellung von Pyridinverbindungen der Formel I,

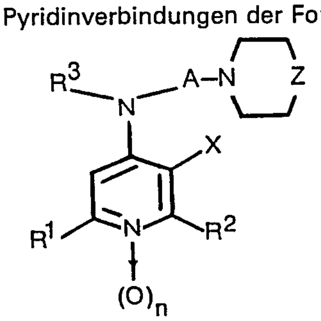

(I)

in der

$R^1$ und $R^2$ unabhängig voneinander für Wasserstoff oder Alkyl mit 1 bis 4 C-Atomen stehen,
$R^3$ Wasserstoff oder Alkyl mit 1 oder 2 C-Atomen und
A geradkettiges oder verzweigtes Alkylen mit 2 bis 4 C-Atomen bedeuten,
n den Wert 0 oder 1 hat,
Z eine Gruppe der Formel Za, Zb oder Zc

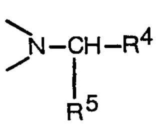

(Za)

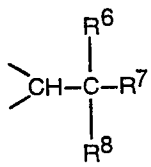

(Zb)

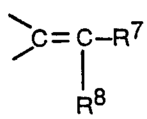

(Zc)

darstellt, wobei entweder
$R^4$ Wasserstoff und
$R^5$ Phenyl oder Cinnamyl oder
$R^4$, $R^5$, $R^7$ und $R^8$ unabhängig voneinander jeweils Pyridyl oder Phenyl, wobei das Phenyl gegebenenfalls bis zu zwei gleiche oder verschiedene Substituenten aus der Gruppe Halogen und Alkoxy mit bis zu 2 C-Atomen trägt,
$R^6$ Wasserstoff oder Hydroxy und
X Wasserstoff, eine Cyan-, Amino- oder Nitrogruppe oder den Rest —CO—$R^9$ bedeutet, in dem $R^9$ für Hydroxy oder Alkoxy mit 1 bis 4 C-Atomen steht,
und die physiologisch verträglichen Salze dieser Verbindungen, dadurch gekennzeichnet, daß man

a) eine Verbindung der Formel II

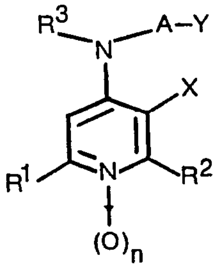

(II)

mit einem cyclischen Amin der Formel III

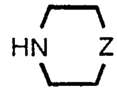

(III)

umsetzt, wobei $R^1$ bis $R^3$, n, X, A und Z mit $R^4$ bis $R^8$ die oben genannten Bedeutungen haben und Y für eine Abgangsgruppe steht, oder

b) eine Verbindung der Formel IV

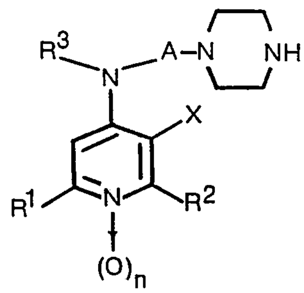

(IV)

mit einer Verbindung der Formel V

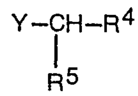

zu einer Verbindung der Formel I mit dem Strukturelement Za umsetzt, wobei $R^1$ bis $R^3$, n, X, A und die voranstehend genannten Bedeutungen haben, oder

c) eine Verbindung der Formel VI

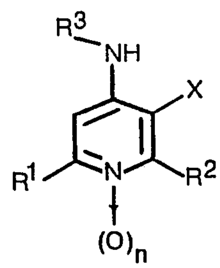

(VI)

mit einem cyclischen Amin der Formel VII

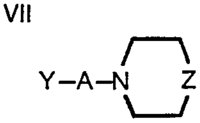

(VII)

umsetzt, wobei $R^1$ bis $R^3$, n, X, A und Z mit $R^4$ bis $R^8$ und Y die voranstehend definierten Bedeutungen haben, oder

d) eine Verbindung der Formel VIII

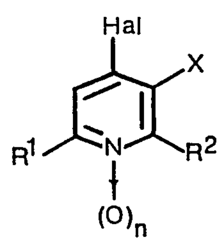

(VIII)

mit einem Amin der Formel IX

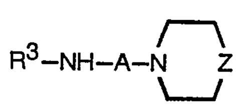

(IX)

umsetzt, wobei $R^1$ bis $R^3$, n, X, A und Z mit $R^4$ bis $R^8$ die oben genannten Bedeutungen haben und Hal ein Halogenatom darstellt,

daß man die Umsetzung gemäß den Verfahrensvarianten a), b), c) und d) in einem gegenüber den Reaktionspartnern inerten Lösungs- oder Verteilungsmittel in Gegenwart eines Amin-Überschusses oder unter Zusatz eines anderen säurebindenden Mittels bei Temperaturen zwischen 0°C und dem Siedepunkt des jeweiligen Reaktionsmediums durchführt und das gemäß diesen Verfahrensvarianten erhaltene Produkt isoliert, wobei man

e) eine Verbindung der Formel I, in der X für die Aminogruppe steht, auch herstellen kann, indem man eine gemäß a), b), c) oder d) erhaltene 3-Nitroverbindung der Formel I mit reduzierend wirkenden Metallsalzen oder durch katalytische Reduktion mit Hydrazin oder Wasserstoff in einem gegenüber den Reaktionsteilnehmern inerten Lösungs- oder Verteilungsmittel bei Temperaturen zwischen 0°C und 100°C bei Atmosphären- oder erhöhtem Druck reduziert, oder

f) eine Verbindung der Formel I, in der X die Carboxylgruppe bedeutet, auch herstellen kann, indem man einen gemäß a), b), c) oder d) erhaltenen 3-Carbonsäureester der Formel I, unter alkalischen Bedingungen, gegebenenfalls bei erhöhter Temperatur, hydrolysiert oder

g) eine Verbindung der Formel I mit dem Strukturmerkmal Zc auch herstellen kann, indem man eine gemäß a), b), c) oder d) erhaltene Verbindung der Formel I mit dem Strukturelement Zb, worin $R^6$ die Bedeutung von Hydroxy hat, in saurem Medium bei Temperaturen zwischen 0°C und dem Siedepunkt des jeweiligen Reaktionsgemisches, dehydratisiert,

wobei man die Verbindungen der Formel I entweder in freier Form isoliert oder sie mit geeigneten Säuren, oder für den Fall, daß X eine Carboxylgruppe darstellt, auch mit geeigneten Basen in physiologisch verträgliche Salze umwandelt.

2. Verfahren nach Anspruch 1, gekennzeichnet durch wenigstens eines der Merkmale, daß die Reste $R^1$ und $R^2$ zusammen nicht mehr als 6 C-Atome enthalten, daß das Halogen, sofern vorhanden, an den Phenylringen Fluor, Chlor oder Brom darstellt und daß die Alkylenbrücke A Äthylen bedeutet.

3. Verfahren nach Anspruch 1 oder 2, dadurch gekennzeichnet, daß in Formel I $R^1$ und $R^2$ zusammen nicht mehr als 2 C-Atome enthalten.

4. Verfahren nach einem oder merheren der Ansprüche 1 bis 3, dadurch gekennzeichnet, daß in Formel I $R^1$ und $R^2$ jeweils Methyl, $R^3$ Wasserstoff, A Äthylen, Z die Gruppe der Formel Za oder Zb und X die Cyan- oder Nitrogruppe bedeuten.

5. Verfahren nach einem oder mehreren der Ansprüche 1 bis 4, dadurch gekennzeichnet, daß in Formel I Z die Gruppe der Formal Za darstellt, in der $R^4$ und $R^5$ die Bedeutung von Phenyl haben, das gegebenenfalls mit bis zu zwei gleichen oder verschiedenen Halogenatomen substituiert ist.

6. Verfahren nach Anspruch 5, dadurch gekennzeichnet, daß es sich um 3-Nitro- oder 3-Cyan-2,6dimethyl-4-[(2-{4-diphenylmethyl-1-piperazinyl}-äthyl)amino]-pyridin bzw. deren 1-Oxide hergestellt werden.

7. Verfahren nach einem oder mehreren der Ansprüche 1 bis 6, gekennzeichnet durch wenigstens eines der Merkmale, daß man die Umsetzung der Pyridinderivate gemäß Formeln II, IV, VI und VIII mit den Verbindungen der Formeln III, V, VII bzw. IX bei Temperaturen zwischen 20 und 100°C durchführt, daß man bei der Reduktion der 3-Nitroverbindungen der Formel I zu den entsprechenden 3-Aminoverbindungen mit katalytisch erregtem Wasserstoff, bei Raumtemperatur und bei Atmosphärendruck arbeitet, daß man die alkalischen Bedingungen für die Hydrolyse der 3-Carbonsäureester der Formel I zu den korrespondierenden Carbonsäuren mit Alkalihydroxiden oder -carbonaten einstellt und daß man die Dehydratisierung der tertiären Alkohole der Formel I mit alkoholischer HCl bei Raumtemperatur vornimmt.

8. Verfahren zur Herstellung einer pharmazeutischen Komposition, die zur Vorbeugung und/oder Behandlung von allergisch konditionierten Erkrankungen, insbesondere allergischer Rhinitis, allergischem Asthma, anaphylaktischem Schock, allergischen Dermatitiden und allergischer Konjunktivitis, geeignet ist, dadurch gekennzeichnet, daß man als aktive Substanz eine Verbindung I einarbeitet, die nach dem Verfahren gemäß einem oder mehreren der Ansprüche 1 bis 7 hergestellt ist, oder ein physiologisch verträgliches Salz davon, in Kombination mit einem physiologisch unbedenklichen Träger derart, daß man eine für eine Verabreichung geeignete Komposition erhält.

9. Verfahren zur herstellung von Arzneimittelformulierungen zur Vorbeugung und/oder Behandlung allergischer Rhinitis, allergischem Asthma, anaphylaktischem Schock, allergischen Dermatitiden und allergischer Konjunktivitis, dadurch gekennzeichnet, daß man eine Verbindung I, die nach dem Verfahren eines oder mehrerer der Ansprüche 1 bis 7 erhalten worden ist, oder ein physiologisch verträgliches Salz davon, mit den üblichen Träger- und Hilfsstoffen zu Arzneimitteln formuliert.

10. Verfahren nach Anspruch 8 oder 9, dadurch gekennzeichnet, daß man feste Dosierungseinheiten herstellt, die bis zu 100 mg der Verbindung I enthalten, oder Injektionslösungen, die bis zu 20 mg der Verbindung I enthalten, wobei die Verbindung I als solche oder in Form eines physiologisch verträglichen Salzes vorliegt.

11. Verfahren nach Anspruch 10, dadurch gekennzeichnet, daß man feste Dosierungseinheiten mit 5—50 mg der Verbindung I und Injektionslösungen mit 1—15 mg der Verbindung I herstellt, wobei die Verbindung I als solche oder in Form eines physiologisch verträglichen Salzes vorliegt.

12. Verbindungen der Formel I oder physiologisch verträgliche Salze davon, die nach dem Verfahren eines oder mehrerer der Ansprüche 1 bis 7 hergestellt worden sind.

**Revendications pour les Etats contractants: BE CH DE FR GB IT LI LU NL SE**

1. Composés de type pyridine de formule I

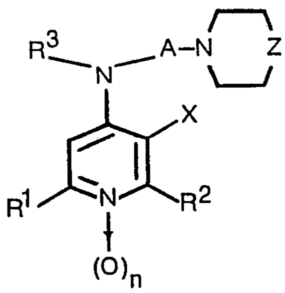

(I)

dans laquelle

$R^1$ et $R^2$ représentent, indépendamment l'un de l'autre, un atome d'hydrogène ou un groupe alkyle ayant de 1 à 4 atomes de carbone,

$R^3$ représente un atome d'hydrogène ou un groupe alkyle ayant 1 ou 2 atomes de carbone, et

A représente un groupe alkylène à chaîne droite ou ramifiée ayant de 2 à 4 atomes de carbone,

n est 0 ou 1,

Z représente un groupe de formule Za, Zb ou Zc

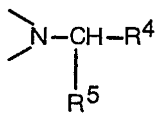 (Za) 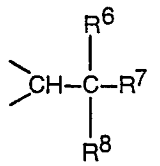 (Zb) 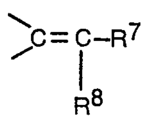 (Zc)

dans lesquelles formules, soit

$R^4$ représente un atome d'hydrogène et

$R^5$ représente le groupe phényle ou cinnamyle, soit

$R^4$, $R^5$, $R^7$ et $R^8$ représentent chacun, indépendamment l'un de l'autre, un radical pyridyle ou phényle, le radical phényle portant éventuellement jusqu'à ou deux substituants identiques ou différents, choisis parmi des atomes d'halogène et des groupes alcoxy ayant jusqu'à deux atomes de carbone,

$R^6$ représente un atome d'hydrogène ou le groupe hydroxy, et

X représente un atome d'hydrogène, le groupe cyano, amino ou nitro, ou un radical —CO—$R^9$, dans lequel $R^9$ représente un groupe hydroxy ou alcoxy ayant de 1 à 4 atomes de carbone, et sels physiologiquement acceptables de ces composés.

2. Composés et leurs sels selon la revendication 1, caractérisés par au moins l'une des caractéristiques que les radicaux $R^1$ et $R^2$ ne contiennent ensemble pas plus de 6 atomes de carbone, que l'halogène sur les noyaux phényle, s'il existe, représente le fluor, le chlore ou le brome, et que le pont alkylène A signifie le groupe éthylène.

3. Composés et leurs sels selon la revendication 1 ou 2, caractérisés en ce que, dans la formule 1, $R^1$ et $R^2$ ne contiennent ensemble pas plus de deux atomes de carbone.

4. Composés et leurs sels selon une ou plusieurs des revendications 1 à 3, caractérisés en ce que, dans la formule I, $R^1$ et $R^2$ représentent chacun le groupe méthyle, $R^3$ représente un atome d'hydrogène, A représente le groupe éthylène, Z représente le groupe de formule Za ou Zb, et X représente le groupe cyano ou nitro.

5. Composés et leurs sels selon une ou plusieurs des revendications 1 à 4, caractérisés en ce que, dans la formule I, Z représente le groupe de formule Za dans lequel $R^4$ et $R^5$ représentent un groupe phényle qui est éventuellement substitué par jusqu'à deux atomes d'halogène identiques ou différents.

6. Composés et leurs sels selon le revendication 5, caractérisés en ce qu'il s'agit de la 3-nitro- ou de la 3-cyano-2,6-diméthyl-4-[(2-{4-diphénylméthyl-1-pipérazinyl}-éthyl)-amino]-pyridine et de leurs oxydes-1.

7. Procédé pour la préparation de composés de type pyridine de formule I selon une ou plusieurs des revendications 1 à 6, et de leurs sels physiologiquement acceptables, caractérisé en ce que

a) on fait réagir un composé de formule II

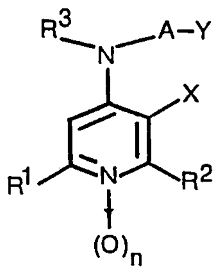

(II)

avec une amine cyclique de formule III

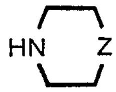

(III)

$R^1$ à $R^3$, n, X, A et Z avec $R^4$ à $R^8$ ayant les significations données dans une ou plusieurs des revendications 1 à 6, et Y représentant un groupe séparable, ou

b) on fait réagir un composé de formule IV

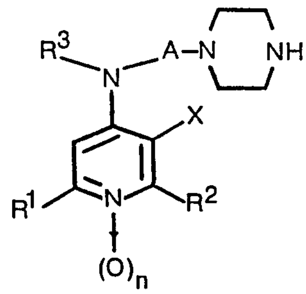

(IV)

avec un composé de formule V

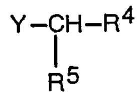

pour aboutir à un composé de formule I comportant l'élément structural Za, $R^1$ à $R^5$, n, X et A ayant les significations définies dans une ou plusieurs des revendications 1 à 6, et Y ayant les significations données précédemment, ou

c) on fait réagir un composé de formule VI

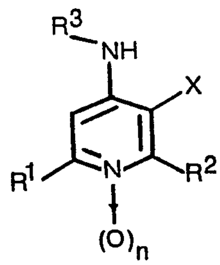

(VI)

avec une amine cyclique de formule VII

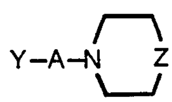

(VII)

$R^1$ à $R^3$, n, X, A et Z avec $R^4$ à $R^8$ ayant les significations données dans une plusieurs des revendications 1 à 6, et Y ayant les significations données précédemment, ou

d) on fait réagir un composé de formule VIII

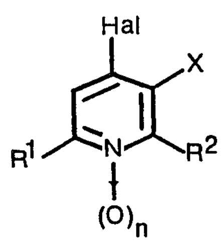

(VIII)

avec une amine de formule IX

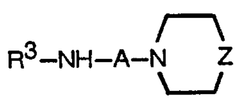

(IX)

$R^1$ à $R^3$, n, X, A et Z avec $R^4$ à $R^8$ ayant les significations données dans une ou plusieurs des revendications 1 à 6, et Hal représentant un atome d'halogène,

en ce que l'on effectue la réaction, selon les variantes a), b), c) et d) du procédé, dans un solvant ou dispersant inerte vis-à-vis des partenaires réactionnels, en présence d'un excès d'amine ou avec addition

d'un autre capteur d'acide, à des températures comprises entre 0°C et le point d'ébullition du mélange réactionnel respectif, et en ce que l'on isole le produit obtenu selon ces variantes, procédé dans lequel

e) on peut également préparer un composé de formule I dans lequel X représente le groupe amino, en réduisant un composé 3-nitro de formule I, obtenu selon a), b), c) ou d), avec des sels métalliques à effet réducteur ou par réduction catalytique avec l'hydrazine ou l'hydrogène, dans un solvant ou dispersant inerte vis-à-vis des partenaires réactionnels, à des températures comprises entre 0 et 100°C, sous une pression égale ou supérieure à la pression atomsphérique, ou

f) on peut également préparer un composé de formule I dans lequel X représente le groupe carboxy, en hydrolysant un ester d'acide 3-carboxylique de formule I, obtenu selon a), b), c) ou d), de préférence dans des conditions alcalines, éventuellement à haute température, ou

g) on peut également préparer un composé de formule I, comportant l'élément structural Zc, en déshydratant un composé de formule I, obtenu selon a), b), c) ou d) et comportant l'élément structural Zb dans lequel $R^6$ représente le groupe hydroxy, en milieu acide, à des températures comprises entre 0°C et le point d'ébullition du mélange réactionnel respectif; et

soit on isole les composés de formule I sous forme libre, soit on les convertit en sels physiologiquement acceptables avec des acides appropriés, ou, dans le cas où X représente un groupe carboxy, également avec des bases appropriées.

8. Procédé selon la revendication 7, caractérisé par au moins l'une des caractéristiques que l'on effectue la réaction des dérivés de pyridine de formules II, IV, VI et VIII avec les composés de formules III, V, VII ou IX à des températures comprises entre 20 et 100°C, en ce que, dans la réduction des composés 3-nitro de formule I conduisant aux composés 3-amino correspondants, on opère avec de l'hydrogène activé par catalyse, à la température ambiante et sous la pression atmosphérique, en ce que l'on ajuste avec des hydroxydes ou carbonates alcalins les conditions alcalines pour l'hydrolyse des esters 3-carboxyliques de formule I conduisant aux acides carboxyliques correspondants, et en ce que l'on effectue la déshydratation des alcools tertiaires de formule I avec de l'HCl en solution alcoolique, à la température ambiante.

9. Médicaments caractérisés par une teneur en — ou consistant en — au moins un composé de formule I et/ou un de ses sels physiologiquement acceptables selon une ou plusieurs des revendications 1 à 6, ou en au moins un composé préparé par le procédé selon la revendication 7 ou 8.

10. Médicaments selon la revendication 9, caractérisés en ce qu'ils sont indiqués pour la prévention et/ou le traitement de maladies causées par des allergies.

11. Médicaments selon la revendication 9 ou 10, caractérisés en ce qu'ils sont indiqués pour la prévention ou le traitement de la rhinite allergique, de l'asthme allergique, du choc anaphylactique, de dermatites allergiques et de la conjonctivite allergique.

12. Dose unitaire d'un médicament selon la revendication 9, 10 ou 11, caractérisé en ce qu'elle contient une quantité efficace d'au moins un composé de formule I, cette dose unitaire se trouvant convenablement soit sous forme de dose unitaire solide contenant jusqu'à 100 mg du composé I, soit sous forme de solution injectable contenant jusqu'à 20 mg du composé I, le composé I se trouvant soit tel quel, soit sous forme d'un sel physiologiquement acceptable.

13. Dose unitaire selon la revendication 12, caractérisée en ce qu'elle contient, en tant que dose unitaire solide, de 5 à 50 mg du composé I, et, en tant que solution injectable, de 1 à 15 mg du composé I, le composé I se trouvant tel quel ou sous forme d'un sel physiologiquement acceptable.

14. Utilisation de composés de type pyridine de formule I et/ou de leurs sels physiologiquement acceptables selon une ou plusieurs des revendications 1 à 6, pour la préparation de médicaments qui sont indiqués pour la prévention et/ou le traitement de maladies causées par des allergies.

**Revendications pour les Etats contractants: GR ES AT**

1. Procédé pour la préparation de composés de type pyridine de formule I

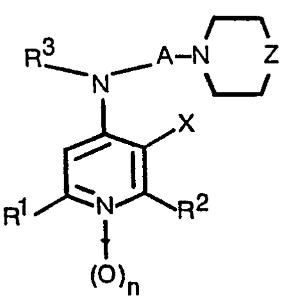

(I)

dans laquelle

$R^1$ et $R^2$ représentent, indépendamment l'un de l'autre, un atome d'hydrogène ou un groupe alkyle ayant de 1 à 4 atomes de carbone,

$R^3$ représente un atome d'hydrogène ou un groupe alkyle ayant 1 ou 2 atomes de carbone, et

A représente un groupe alkylène à chaîne droite ou ramifiée ayant de 2 à 4 atomes de carbone,

n est 0 ou 1,

Z représente un groupe de formule Za, Zb ou Zc

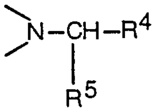 (Za) 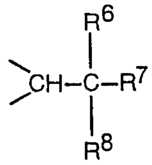 (Zb) 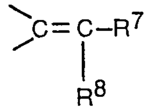 (Zc)

dans lesquelles formules, soit

$R^4$ représente un atome d'hydrogène et

$R^5$ représente le groupe phényle ou cinnamyle, soit

$R^4$, $R^5$, $R^7$ et $R^8$ représentent chacun, indépendamment l'un de l'autre, un radical pyridyle ou phényle, le radical phényle portant éventuellement jusqu'à ou deux substituants identiques ou différents, choisis parmi des atomes d'halogène et des groupes alcoxy ayant jusqu'à deux atomes de carbone,

$R^6$ représente un atome d'hydrogène ou le groupe hydroxy, et

X représente un atome d'hydrogène, le groupe cyano, amino ou nitro, ou un radical —CO—$R^9$, dans lequel $R^9$ représente un groupe hydroxy ou alcoxy ayant de 1 à 4 atomes de carbone, et des sels physiologiquement acceptables de ces composés, caractérisé en ce que

a) on fait réagir un composé de formule II

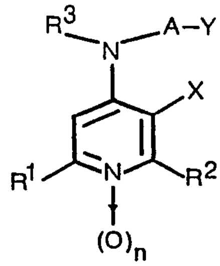

(II)

avec une amine cyclique de formule III

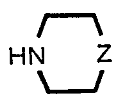

(III)

$R^1$ à $R^3$, n, X, A et Z avec $R^4$ à $R^8$ ayant les significations données plus haut, et Y représentant un groupe séparable, ou

b) on fait réagir un composé de formule IV

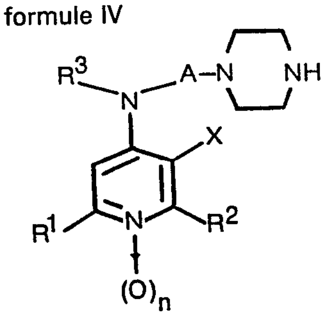

(IV)

avec un composé de formule V

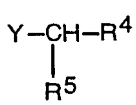

pour aboutir à un composé de formule I comportant l'élément structural Za, $R^1$ à $R^5$, n, X, A et Y ayant les significations données précédemment, ou

c) on fait réagir un composé de formule VI

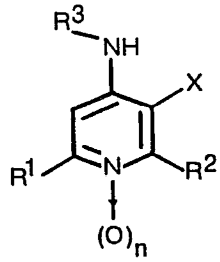

(VI)

avec une amine cyclique de formule VII

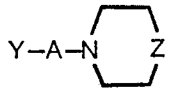

(VII)

$R^1$ à $R^3$, n, X, A et Z avec $R^4$ à $R^8$, et Y ayant les significations données précédemment, ou

d) on fait réagir un composé de formule VIII

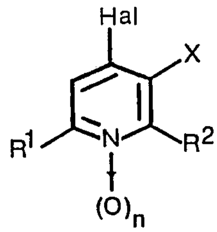

(VIII)

avec une amine de formule IX

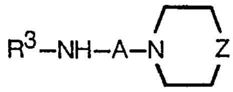

(IX)

$R^1$ à $R^3$, n, X, A et Z avec $R^4$ à $R^8$ ayant les significations données précédemment et Hal représentant un atome d'halogène,

en ce que l'on effectue la réaction, selon les variantes a), b), c) et d) du procédé, dans un solvant ou dispersant inerte vis-à-vis des partenaires réactionnels, en présence d'un excès d'amine ou avec addition d'un autre capteur d'acide, à des températures comprises entre 0°C et le point d'ébullition du mélange réactionnel respectif, et en ce que l'on isole le produit obtenu selon ces variantes, procédé dans lequel

e) on peut également préparer un composé de formule I dans lequel X représente le groupe amino, en réduisant un composé 3-nitro de formule I, obtenu selon a), b), c) ou d), avec des sels métalliques à effet réducteur ou par réduction catalytique avec l'hydrazine ou l'hydrogène, dans un solvant ou dispersant inerte vis-à-vis des partenaires réactionnels, à des températures comprises entre 0 et 100°C, sous une pression égale ou supérieure à la pression atmosphérique, ou

f) on peut également préparer un composé de formule I dans lequel X représente le groupe carboxy, en hydrolysant un ester d'acide 3-carboxylique de formule I, obtenu selon a), b), c) ou d), dans des conditions alcalines, éventuellement à haute température, ou

g) on peut également préparer un composé de formule I, comportant l'élément structural Zc, en déshydratant un composé de formule I, obtenu selon a), b), c) ou d) et comportant l'élément structural Zb dans lequel $R^6$ représente le groupe hydroxy, en milieu acide, à des températures comprises entre 0°C et le point d'ébullition du mélange réactionnel respectif; et

soit on isole les composés de formule I sous forme libre, soit on les convertit en sels physiologiquement acceptables avec des acides appropriés, ou, dans le cas où X représente un groupe carboxy, également avec des bases appropriées.

2. Procédé selon la revendication 1, caractérisé par au moins l'une des caractéristiques que les radicaux $R^1$ et $R^2$ ne contiennent ensemble pas plus de 6 atomes de carbone, que l'halogène sur les noyaux phényle, s'il existe, représente le fluor, le chlore ou le brome, et que le pont alkylène A signifie le groupe éthylène.

3. Procédé selon la revendication 1 ou 2, caractérisé en ce que, dans la formule 1, $R^1$ et $R^2$ ne contiennent ensemble pas plus de deux atomes de carbone.

4. Procédé selon une ou plusieurs des revendications 1 à 3, caractérisé en ce que, dans la formule I, $R^1$ et $R^2$ représentent chacun le groupe méthyle, $R^3$ représente un atome d'hydrogène, A représente le groupe éthylène, Z représente le groupe de formule Za ou Zb, et X représente le groupe cyano ou nitro.

5. Procédé selon une ou plusieurs des revendications 1 à 4, caractérisé en ce que, dans la formule I, Z représente le groupe de formule Za dans lequel $R^4$ et $R^5$ représentent un groupe phényle qui est éventuellement substitué par jusqu'à deux atomes d'halogène identiques ou différents.

6. Procédé selon la revendication 5, caractérisé en ce que l'on prépare la 3-nitro- ou la 3-cyano-2,6-diméthyl-4-[(2-{4-diphénylméthyl-1-pipérazinyl}-éthyl)-amino]-pyridine ou leurs oxydes-1.

7. Procédé selon une ou plusieurs des revendications 1 à 6, caractérisé par au moins l'une des caractéristiques que l'on effectue la réaction des dérivés de pyridine de formules II, IV, VI et VIII avec les composés de formules III, V, VII ou IX à des températures comprises entre 20 et 100°C, en ce que, dans la réduction des composés 3-nitro de formule I conduisant aux composés 3-amino correspondants, on opère avec de l'hydrogène activé par catalyse, à la température ambiante et sous la pression atmosphérique, en ce que l'on ajuste avec des hydroxydes ou carbonates alcalins les conditions alcalines pour l'hydrolyse des esters 3-carboxyliques de formule I conduisant aux acides carboxyliques correspondants, et en ce que l'on effectue la déshydratation des alcools tertiaires de formule I avec de l'HCl en solution alcoolique, à la température ambiante.

8. Procédé pour la préparation d'une composition pharmaceutique qui est appropriée à la prévention et/ou au traitement de maladies causées par des allergie, en particulier de la rhinite allergique, de l'asthme allergique, du choc anaphylactique, de dermatites allergiques et de la conjonctivite allergique, caractérisé en ce que l'on incorpore en tant que substance active un composé I qui est préparé par le procédé selon une ou plusieurs des revendications 1 à 7, ou un sel physiologiquement acceptable de celui-ci, en association avec un véhicule physiologiquement acceptable, de manière à obtenir une composition appropriée à l'administration.

9. Procédé pour la fabrication de formulations de médicaments pour la prévention et/ou le traitement de la rhinite allergique, de l'asthme allergique, du choc anaphylactique, de dermatites allergiques et de la conjonctivite allergique, caractérisé en ce que l'on formule en médicaments un composé I qui a été obtenu par le procédé d'une ou plusieurs des revendications 1 à 7, ou un sel physiologiquement acceptable de celui-ci, avec les véhicules et produits auxiliaires usuels.

10. Procédé selon la revendication 8 ou 9, caractérisé en ce que l'on prépare des doses unitaires solides qui contiennent jusqu'à 100 mg du composé I, ou des solutions injectables qui contiennent jusqu'à 20 mg du composé I, le composé I se trouvant tel quel ou sous forme d'un sel physiologiquement acceptable.

11. Procédé selon la revendication 10, caractérisé en ce que l'on prépare des doses unitaires solides contenant de 5 à 50 mg du composé I, et des solutions injectables contenant de 1 à 15 mg du composé I, le composé I se trouvant tel quel ou sous forme d'un sel physiologiquement acceptable.

12. Composés de formule I ou sels physiologiquement acceptables de ceux-ci, qui ont été préparés par le procédé d'une ou plusieurs des revendications 1 à 7.

**Claims for the Contracting States: BE CH DE FR GB IT LI LU NL SE**

1. A pyridine compound of the formula I

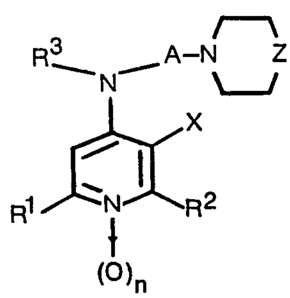

(I)

in which

$R^1$ and $R^2$, independently of one another, represent hydrogen or alkyl having 1 to 4 carbon atoms,
$R^3$ denotes hydrogen or alkyl having 1 or 2 carbon atoms, and
A denotes straight-chain or branched alkylene having 2 to 4 carbon atoms,
n has the value 0 or 1,
Z represents a group of the formula Za, Zb or Zc

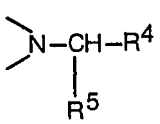 (Za) 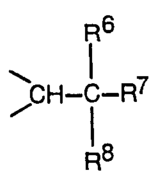 (Zb) 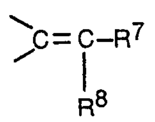 (Zc)

either
$R^4$ denoting hydrogen and
$R^5$ denoting phenyl or cinnamyl or
$R^4$, $R^5$, $R^7$ and $R^8$, independently of one another, each denoting pyridyl or phenyl, the phenyl optionally carrying one or two identical or different substituents from the group comprising halogen and alkoxy having 1 or 2 carbon atoms,
$R^6$ denoting hydrogen or hydroxy, and
X denoting hydrogen, a cyano, amino or nitro group or the radical —CO—$R^9$, in which $R^9$ represents hydroxyl or alkoxy having 1 to 4 carbon atoms, and the physiologically tolerated salts of this compound.

2. A compound and its salts as claimed in claim 1, which has at least one of the following features: the radicals $R^1$ and $R^2$ together contain not more than 6 carbon atoms, the halogen, where present, on the phenyl rings represents fluorine, chlorine or bromine, and the alkylene bridge A denotes ethylene.

3. A compound and its salts as claimed in claim 1 or 2, with $R^1$ and $R^2$ in formula I together containing not more than 2 carbon atoms.

4. A compound and its salts as claimed in one or more of claims 1 to 3, with, in formula I, $R^1$ and $R^2$ each denoting methyl, $R^3$ denoting hydrogen, A denoting ethylene, Z denoting the group of the formula Za or Zb and X denoting the cyano or nitro group.

5. A compound and its salts as claimed in one or more of claims 1 to 4, with Z in formula I representing the group of the formula Za in which $R^4$ and $R^5$ have the meaning of phenyl which is optionally substituted by up to two identical or different halogen atoms.

6. A compound and its salts as claimed in claim 5, which is 3-nitro- or 3-cyano-2,6-dimethyl-4-[(2-{4-diphenylmethyl-1-piperazinyl}ethyl)amino]pyridine and their 1-oxides.

7. A process for the preparation of pyridine compounds of the formula I as claimed in one or more of claims 1 to 6 and of their physiologically tolerated salts, which comprises

a) reaction of a compound of the formula II

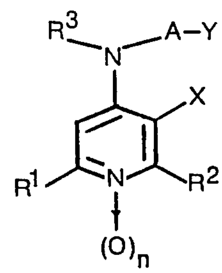

(II)

with a cyclic amine of the formula III

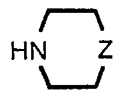

(III)

$R^1$ to $R^3$, n, X, A and Z with $R^4$ and $R^8$ having the meanings mentioned in one or more of claims 1 to 6, and Y representing a leaving group, or

b) reaction of a compound of the formula IV

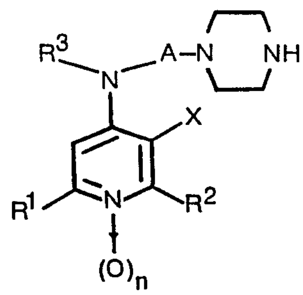

(IV)

with a compound of the formula V

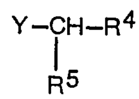

to give a compound of the formula I having the structural element Za, with $R^1$ to $R^5$, n, X and A having the meanings defined in one or more of claims 1 to 6, and Y having the abovementioned meanings, or

c) reaction of a compound of the formula VI

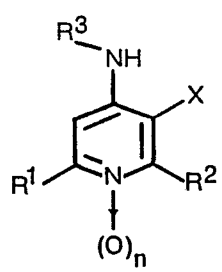

(VI)

with a cyclic amine of the formula VII

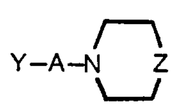

(VII)

$R^1$ to $R^3$, n, X, A and Z with $R^4$ to $R^8$ having the meanings defined in one or more of claims 1 to 6, and Y having the meanings defined above, or

d) reaction of a compound of the formula VIII

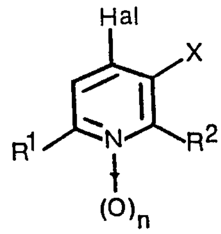

(VIII)

with an amine of the formula IX

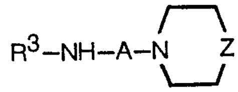

(IX)

$R^1$ to $R^3$, n, X, A and Z with $R^4$ to $R^8$ having the meanings mentioned in one or more of claims 1 to 6, and Hal representing a halogen atom,
reaction being effected by process variants a), b), c) and d) in a solvent or dispersant which is inert towards the reactants, in the presence of an excess of amine or with the addition of another acid-binding agent, at temperatures between 0°C and the boiling point of the particular reaction medium, and isolation of the product obtained by these process variants, it also being possible

e) to prepare a compound of the formula I in which X represents the amino group by reduction of a 3-nitro compound of the formula I obtained as in a), b), c) or d) with metal salts having a reducing action or by catalytic reduction with hydrazine or hydrogen in a solvent or dispersant which is inert towards the reactants, at temperatures between 0°C and 100°C under atmospheric or elevated pressure, or

f) to prepare a compound of the formula I in which X denotes the carboxyl group by hydrolysis of a 3-carboxylic ester of the formula I obtained as in a), b), c) or d), preferably under alkaline conditions, where appropriate at elevated temperature, or

g) to prepare a compound of the formula I having the structural feature Zc by dehydration of a compound of the formula I which has the structural element Zb, in which $R^6$ has the meaning of hydroxyl, and which has been obtained as in a), b), c) or d), in acid medium at temperatures between 0°C and the boiling point of the particular reaction mixture,
the compounds of the formula I either being isolated in free form or being converted with suitable acids or, in the case where X represents a carboxyl group, with suitable bases into physiologically tolerated salts.

8. The process as claimed in claim 7, which has at least one of the following features: the reaction of the pyridine derivatives of formulae II, IV, VI and VIII with the compounds of the formulae III, V, VII and IX respectively is carried out at temperatures between 20° and 100°C, the reduction of the 3-nitro compounds of the formula I to give the corresponding 3-amino compounds is carried out with catalytically activated hydrogen at room temperature and under atmospheric pressure, the alkaline conditions for the hydrolysis of the 3-carboxylic esters of the formula I to give the corresponding carboxylic acids are set up with alkali metal hydroxides or carbonates and the dehydration of the tertiary alcohols of the formula I is carried out with alcoholic HCl at room temperature.

9. A medicament which contains, or is composed of, at least one compound of the formula I and/or one of its physiologically tolerated salts as claimed in one or more of claims 1 to 6, or at least one compound prepared by the process as claimed in claims 7 or 8.

10. A medicament as claimed in claim 9, which is intended for the prevention and/or treatment of allergically conditioned disorders.

11. A medicament as claimed in claim 9 or 10, which is intended for the prevention and/or treatment of allergic rhinitis, allergic asthma, anaphylactic shock, allergic dermatitis and allergic conjunctivitis.

12. A dosage unit of a medicament as claimed in claim 9, 10 or 11, which contains an effective amount of at least one compound of the formula I, this dosage unit advantageously being in the form either of a solid dosage unit which contains up to 100 mg of the compound I or of an injection solution which contains up to 20 mg of the compound I, the compound I being present as such or in the form of a physiologically tolerated salt.

13. A dosage unit as claimed in claim 12, which contains 5—50 mg of the compound I when in the form of a solid dosage unit and 1—15 mg of the compound I when in the form of an injection solution, the compound I being present as such or in the form of a physiologically tolerated salt.

14. The use of pyridine compounds of the formula I and/or of their physiologically tolerated salts as claimed in one or more of claims 1—6 for the preparation of medicaments which are intended for the prevention and/or treatment of allergically conditioned disorders.

**Claims for the Contracting States: GR ES AT**

1. A process for the preparation of pyridine compounds of the formula I

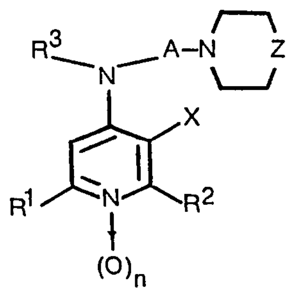

(I)

in which
$R^1$ and $R^2$, independently of one another, represent hydrogen or alkyl having 1 to 4 carbon atoms,
$R^3$ denotes hydrogen or alkyl having 1 or 2 carbon atoms, and
A denotes straight-chain or branched alkylene having 2 to 4 carbon atoms,
n has the value 0 or 1,
Z represents a group of the formula Za, Zb or Zc

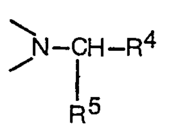 (Za) 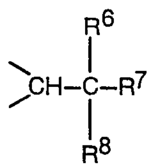 (Zb) 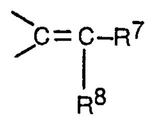 (Zc)

either
$R^4$ denoting hydrogen and
$R^5$ denoting phenyl or cinnamyl or
$R^4$, $R^5$, $R^7$ and $R^8$, independently of one another, each denoting pyridyl or phenyl, the phenyl optionally carrying one or two identical or different substituents from the group comprising halogen and alkoxy having 1 or 2 carbon atoms,
$R^6$ denoting hydrogen or hydroxy, and
X denoting hydrogen, a cyano, amino or nitro group or the radical —CO—$R^9$, in which $R^9$ represents hydroxyl or alkoxy having 1 to 4 carbon atoms, and the physiologically tolerated salts of this compound, which comprises
a) reaction of a compound of the formula II

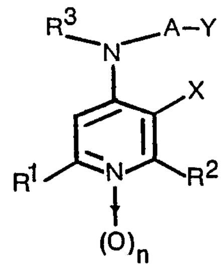

(II)

with a cyclic amine of the formula III

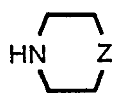

(III)

$R^1$ to $R^3$, n, X, A and Z with $R^4$ and $R^8$ having the meanings mentioned above, and Y representing a leaving group, or
b) reaction of a compound of the formula IV

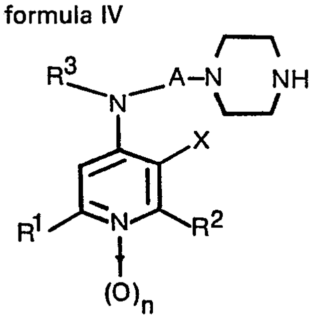

(IV)

with a compound of the formula V

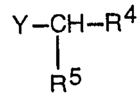

to give a compound of the formula I having the structural element Za, with $R^1$ to $R^5$, n, X, A and Y having the above mentioned meanings, or

c) reaction of a compound of the formula VI

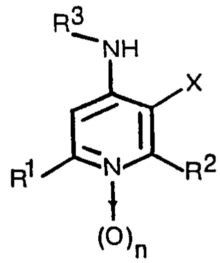

(VI)

with a cyclic amine of the formula VII

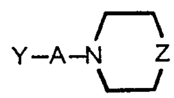

(VII)

$R^1$ to $R^3$, n, X, A and Z with $R^4$ to $R^8$ and Y having the meanings defined above, or

d) reaction of a compound of the formula VIII

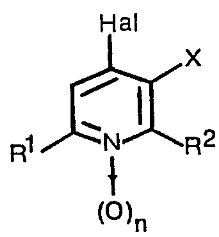

(VIII)

with an amine of the formula IX

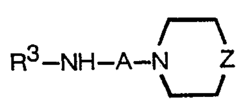

(IX)

$R^1$ to $^3$, n, X, A and Z with $R^4$ and $R^8$ having the meanings mentioned above, and Hal representing a halogen atom,
reaction being effected by process variants a), b), c) and d) in a solvent or dispersant which is inert towards the reactants, in the presence of an excess of amine or with the addition of another acid-binding agent, at temperatures between 0°C and the boiling point of the particular reaction medium, and isolation of the product obtained by these process variants, it also being possible

e) to prepare a compound of the formula I in which X represents the amino group by reduction of a 3-nitro compound of the formula I obtained as in a), b), c) or d) with metal salts having a reducing action or by catalytic reduction with hydrazine or hydrogen in a solvent or dispersant which is inert towards the reactants, at temperatures between 0°C and 100°C under atmospheric or elevated pressure, or

f) to prepare a compound of the formula I in which X denotes the carboxyl group by hydrolysis of a 3-carboxylic ester of the formula I obtained as in a), b), c) or d), under alkaline conditions, where appropriate at elevated temperature, or

g) to prepare a compound of the formula I having the structural feature Zc by dehydration of a compound of the formula I which has the structural element Zb, in which $R^6$ has the meaning of hydroxyl, and which has been obtained as in a), b), c) or d), in acid medium at temperatures between 0°C and the boiling point of the particular reaction mixture,
the compounds of the formula I either being isolated in free form or being converted with suitable acids or, in the case where X represents a carboxyl group, with suitable bases into physiologically tolerated salts.

2. The process as claimed in claim 1 which has at least one of the following features: the radicals $R^1$ and $R^2$ together contain not more than 6 carbon atoms, the halogen, where present, on the phenyl rings represents fluorine, chlorine or bromine, and the alkylene bridge A denotes ethylene.

3. The process as claimed in claim 1 or 2, with $R^1$ and $R^2$ in formula I together containing not more than 2 carbon atoms.

4. The process as claimed in one or more of claims 1 to 3, with, in formula I, $R^1$ and $R^2$ each denoting methyl, $R^3$ denoting hydrogen, A denoting ethylene, Z denoting the group of the formula Za or Zb and X denoting the cyano or nitro group.

5. The process as claimed in one or more of claims 1 to 4, with Z in formula I representing the group of the formula Za in which $R^4$ and $R^5$ have the meaning of phenyl which is optionally substituted by up to two identical or different halogen atoms.

6. The process as claimed in claim 5, in which is prepared 3-nitro- or 3-cyano-2,6-dimethyl-4-[(2-{4-diphenylmethyl-1-piperazinyl}ethyl)amino]pyridine or their 1-oxides.

7. The process as claimed in one or more of claims 1 to 6, which has at least one of the following features: the reaction of the pyridine derivatives of formulae II, IV, VI and VIII with the compounds of the formulae III, V, VII and IX respectively is carried out at temperatures between 20° and 100°C, the reduction of the 3-nitro compounds of the formula I to give the corresponding 3-amino compounds is carried out with catalytically activated hydrogen at room temperature and under atmospheric pressure, the alkaline conditions for the hydrolysis of the 3-carboxylic esters of the formula I to give the corresponding carboxylic acids are set up with alkali metal hydroxides or carbonates, and the dehydration of the tertiary alcohols of the formula I is carried out with alcoholic HCl at room temperature.

8. A process for the preparation of a pharmaceutical composition which is suitable for the prevention and/or treatment of allergically conditioned disorders, in particular allergic rhinitis, allergic asthma, anaphylatic shock, allergic dermatitis and allergic conjunctivitis, which comprises incorporating as active substance a compound I which is prepared by the process as claimed in one or more of claims 1 to 7, or a physiologically tolerated salt thereof, in combination with a physiologically acceptable vehicle in such a way that a composition suitable for administration is obtained.

9. A process for the preparation of pharmaceutical formulations for the prevention and/or treatment of allergic rhinitis, allergic asthma, anaphylactic shock, allergic dermatitis and allergic conjunctivitis, which comprises formulating a compound I which has been obtained by the process of one or more of claims 1 to 7, or a physiologically tolerated salt thereof, with the customary excipients and auxiliaries to provide pharmaceuticals.

10. A process as claimed in claim 8 or 9, in which is prepared a solid dosage unit which contains up to 100 mg of the compound I, or an injection solution which contains up to 20 mg of the compound I, the compound I being present as such or in the form of a physiologically tolerated salt.

11. The process as claimed in claim 10, in which is prepared a solid dosage unit with 5—50 mg of the compound I, and an injection solution with 1—15 mg of the compound I, the compound I being present as such or in the form of a physiologically tolerated salt.

12. A compound of the formula I or physiologically tolerated salts thereof, which have been prepared by the process of one or more of claims 1 to 7.